(19) Europäisches Patentamt
European Patent Office
Office européen des brevets

(11) Veröffentlichungsnummer: **0 291 809 B1**

(12) **EUROPÄISCHE PATENTSCHRIFT**

(45) Veröffentlichungstag der Patentschrift: **15.01.92**

(51) Int. Cl.$^5$: **C07D 215/38**, C07D 215/54, A01N 43/42

(21) Anmeldenummer: **88107411.6**

(22) Anmeldetag: **07.05.88**

(54) **2-Anilino-chinoline, Verfahren zu ihrer Herstellung und diese enthaltende Fungizide.**

(30) Priorität: **16.05.87 DE 3716512**

(43) Veröffentlichungstag der Anmeldung:
**23.11.88 Patentblatt 88/47**

(45) Bekanntmachung des Hinweises auf die Patenterteilung:
**15.01.92 Patentblatt 92/03**

(84) Benannte Vertragsstaaten:
**AT BE CH DE ES FR GB GR IT LI NL SE**

(56) Entgegenhaltungen:

**CHEMICAL ABSTRACTS, Band 72, Nr. 17, 27 April 1970, Columbus, OH (US); HEINDEL et al., Seite 370, Nr. 90236v**

**CHEMICAL ABSTRACTS, Band 69, Nr. 23, 02 Dezember 1968, Columbus, OH (US); Seite 9042, Nr. 96598z**

**JOURNAL MEDICINAL CHEMISTRY, 1970, Nr. 13(1); Seiten 166-167**

**GAZZETTA CHIMICA ITAL., 1968, Nr. 95(5); Seiten 511-534**

(73) Patentinhaber: **BASF Aktiengesellschaft**
**Carl-Bosch-Strasse 38**
**W-6700 Ludwigshafen(DE)**

(72) Erfinder: **Schubert, Jürgen, Dr.**
**Am Oberen Luisenpark 2**
**W-6800 Mannheim 1(DE)**
Erfinder: **Wild, Jochen, Dr.**
**An der Marlach**
**W-6705 Deidesheim(DE)**
Erfinder: **Roeser, Karl, Dr.**
**Muldweg 11**
**W-6945 Hirschberg(DE)**
Erfinder: **Sauter, Hubert, Dr.**
**Neckarpromenade 20**
**W-6800 Mannheim 1(DE)**
Erfinder: **Pommer, Ernst-Heinrich, Dr.**
**Berliner Platz 7**
**W-6703 Limburgerhof(DE)**
Erfinder: **Ammermann, Eberhard, Dr.**
**Sachsenstr. 3**
**W-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft neue, wertvolle 2-Anilino-chinoline, Verfahren zu ihrer Herstellung, diese Verbindungen enthaltende fungizide Mittel und ihre Verwendung als Fungizide.

Es ist bekannt, daß 2-Anilino-pyridine, zum Beispiel das 2-(2,4-Dinitro-6-trifluormethylanilino)-5-trifluormethylpyridin (EP 31 257) eine gute fungizide Wirkung zeigen. Die Wirkung ist jedoch bei niedrigen Aufwandmengen und Anwendungskonzentrationen nicht immer befriedigend.

Es sind ferner 2-Anilino-chinoline bekannt, von denen keine fungizide Wirkung beschrieben wird (C.A. 72, 90236 v (1970) und C.A. 69, 96598 z (1968)).

Überraschend wurde nun gefunden, daß 2-Anilino-chinoline der Formel I

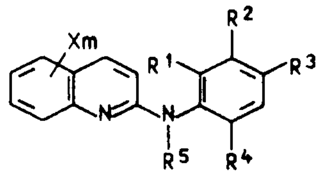

in welcher

X für Wasserstoff, $NO_2$, CN, Hal, $SO_2$-$C_1$-$C_4$-Alkyl, $C_1$-$C_4$-Alkyl, $C_1$-$C_4$-Haloalkyl steht,

m eine ganze Zahl von 1 - 6 ist, wobei die einzelnen Reste gleich oder verschieden sind, wenn m größer als 1 ist,

$R^1$, $R^3$, $R^4$ unabhängig voneinander Wasserstoff, $NO_2$, Hal, CN, $C_1$-$C_6$-Alkyl, $SO_2$-$C_1$-$C_4$-Alkyl, $C_1$-$C_6$-Haloalkyl, $SO_2NR^6R^7$,

$R^2$ Wasserstoff, Hal, $C_1$-$C_4$-Haloalkyl ist,

$R^5$ Wasserstoff, $C_1$-$C_4$-Alkyl, CHO, $COR^8$, $SO_2R^8$, $C_3$-$C_6$-Alkenyl, $C_3$-$C_6$-Alkinyl, Benzyl,

$R^6$, $R^7$ unabhängig voneinander Wasserstoff, $C_1$-$C_4$-Alkyl ist,

$R^8$ $C_1$-$C_4$-Alkyl, $C_1$-$C_4$-Haloalkyl, Benzyl, gegebenenfalls subst. Aryl ist, und Salze dieser 2-Anilino-chinoline der Formel I, in der $R^5$ Wasserstoff bedeutet, mit pflanzenverträglichen Kationen eine sehr gute fungizide Wirkung bei ausgezeichneter Vertraglichkeit bei Pflanzen haben.

Unter dem Begriff Alkyl selbst oder als Bestandteil eines anderen Substituenten, wie Haloalkyl und $SO_2$-Alkyl sind je nach Zahl der angegebenen Kohlenstoffatome die folgenden geradkettigen oder verzweigten Gruppen zu verstehen: Methyl, Ethyl, n-Propyl, iso-Propyl, n-Butyl, sec-Butyl, iso-Butyl, tert.-Butyl, n-Pentyl, n-Hexyl. Die Vorsilbe Halo in der Bezeichnung eines Substituenten bedeutet hier und im folgenden, daß dieser Substituent einfach bis perhalogeniert auftreten kann. Hal und Halo in der $R^1$, $R^2$, $R^3$, $R^4$ die in Anspruch 1 angegebene Bedeutung haben, in Substanz oder in einem Lösungs- oder Verdünnungsmittel, gegebenenfalls in Anwesenheit einer organischen oder anorganischen Base bei Temperaturen zwischen -20 und 200 °C umsetzt, wobei Y und Z für $NH_2$ oder Halogen stehen und Y und Z jeweils voneinander verschieden sind.

In beiden Fällen erfolgt die Umsetzung unter Abspaltung von Halogenwasserstoff. In beiden Fällen ist deshalb die Verwendung von säurebindenden Mitteln bzw. Basen von Vorteil.

Als Basen bzw. säurebindende Mittel können organische und anorganische Verbindungen verwendet werden, wie z.B. Alkali- und Erdalkalihydroxide stehen stellvertretend für F, Cl, Br oder J. Haloalkyl steht somit für einen einfach bis perhalogenieiten Alkylrest, wie $CH_2Cl$, $CHCl_2$, $CCl_3$, $CH_2F$, $CHF_2$, $CF_3$, $CH_2Br$, $CHBrCl$, $CF_2Cl$, $C_2Cl_5$, $C_2F_5$, $CF_2$-$CHF_2$, $CH_2CH_2Cl$, $CH_2CH_2Br$, $C_3F_7$, $C_4F_9$.

Alkenyl steht z.B. für Allyl, 3-Butenyl, 4-Butenyl, trans-2-Butenyl, cis-2-Butenyl.

Alkinyl steht z.B. für Propargyl, 3-Butinyl, 4-Butinyl, 5-Pentinyl.

Gegebenenfalls substituiertes Aryl steht z.B. für Phenyl, Tolyl, 2-, 3-, 4-Tolyl, Halogenphenyl, 4-Bromphenyl, 4-Chlor-phenyl, 4-Fluor-phenyl, Nitrophenyl, 4-Nitrophenyl, 1- und 2-Naphthyl.

Für die Salze kommen beispielsweise folgende Kationen in Betracht: $Na^+$, $K^+$, $Mg^{2+}$, $Fe^{3+}$, $MH_4^+$, einfach oder mehrfach alkylierte und/oder arylierte Ammoniumkationen, wie Diisopropylammonium. Tetramethylammonium, Tetrabutylammonium, Trimethylbenzylammonium, N,N-Dimethylanilinium.

Der Rest $X_m$ kann sowohl im Phenylteil als auch im Pyridinylteil des Chinolinsystems stehen.

Bevorzugt werden die Verbindungen 2-(2,4-Dinitro-6-trifluormethylanilino)-3-cyanochinolin, 2-(2,6-Dinitro-3-chlor-4-trifluormethylanilino)-3-cyano-chinolin, die Verbindungen, in denen

$X_m$ den Cyanrest in 3-Stellung, $R^1$ den Nitrorest, $R^2$ Wasserstoff oder Halogen und $R^4$ Nitro oder Trifluormethyl bedeuten oder

$X_m$ den Cyanrest in 3-Stellung, $R^1$ den Nitrorest, $R^2$ Wasserstoff oder Halogen und $R^3$ den Nitrorest bedeuten.

Ferner werden bevorzugt die Verbindungen mit $X_m$ 3-CN, $R^5$ H, $R^1$ $NO_2$, $R^2$ H, Halogen, Cl, $R^3$ $NO_2$, $CF_3$, $R^4$ $NO_2$, $CF_3$ und die Verbindungen mit $X_m$ 3-CN, $R^1$ $NO_2$, $R^2$ H oder Halogen und $R^3$ Nitro oder $R^4$ Nitro.

Die Verbindungen der Formel I gemäß Anspruch 1, in der $R^5$ Wasserstoff ist, werden z.B. dadurch hergestellt, daß man ein Chinolin der Formel II, in der X und m die in Anspruch 1 angegebene Bedeutung haben, mit Verbindungen der Formel III

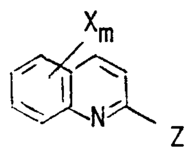

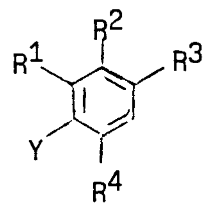

,

II III

(LiOH, NaOH, KOH, Ca(0H)2, usw.), -oxide ($Na_2O$, $Li_2O$, CaO, MgO usw.) -hydride (LiH, NaH, KH, $CaH_2$ usw.), -amide ($LiNH_2$, $NaNH_2$, $KNH_2$, usw.) -carbonate ($Li_2CO_3$, $Na_2CO_3$, $CaCO_3$ usw.), -hydrogencarbonate ($NaHCO_3$ usw.), -alkyle (BuLi, $CH_3Li$, $CH_3MgCl$, usw.), -$C_1$-$C_5$-alkoholate ($NaOCH_3$, $NaOCH_3$, $KOC_2H_5$, KO tert.-Butyl, $Mg(OCH_3)_2$ usw.), Amine, insbes. tertiäre Amine (z.B. Trimethylamin, Triethylamin, Diisopropylethylamin, N-Methylpiperidin usw.), Pyridin, substituierte Pyridine (Collidin, Lutidine, 4-Dimethylaminopyridin usw.), bicyclische Amine.

Die Reaktionen können in Anwesenheit von reaktionsinerten Lösungs- oder Verdünnungsmitteln durchgeführt werden.

In Frage kommen beispielsweise aliphatische und aromatische Kohlenwasserstoffe wie Toluol, Xylole, Cyclohexan, Petrolether; halogenierte Kohlenwasserstoffe wie Chlorbenzol, Methylenchlorid, Chloroform; Ether und etherartige Verbindungen wie Dialkylether (Diethylether Diisopropylether, tert.-Butylmethylether usw.), Anisol, Dioxan, Tetrahydrofuran; Nitrile wie Acetonitril, Propionitril, N,N-dialkylierte Amide wie Dimethylformamid; Dimethylsulfoxid; Ketone wie Aceton, Diethylketon, Methylethylketon, Alkohole wie Methanol, Ethanol, n-und iso-Propanol, Butanole, insbes. tert.-Butanol und Gemische solcher Lösungsmittel untereinander.

Die Reaktionstemperatur läßt sich in weiten Grenzen ändern. Je nach Art der Substituenten X, $R^1$, $R^2$, $R^3$, $R^4$, $R^6$, $R^7$ und ihrer Zahl m arbeitet man vorteilhaft bei Temperaturen zwischen -20 °C und 200 °C bzw. am Siedepunkt des Lösungsmittels bzw. Lösungsmittelgemisches.

In manchen Fällen kann es auch von Vorteil sein, die Reaktion unter Schutzgasatmosphäre und/oder absoluten Lösungsmitteln durchzuführen. Als Schutzgas eignen sich inerte Gase wie Helium, Argon usw., bevorzugt Stickstoff.

Die Ausgangsverbindungen sind im Handel erhältlich, bekannt oder nach gängigen Methoden einfach herstellbar. Die eingesetzten Halogenaromaten (bzw. einfach in diese umwandelbare Phenole) und Aniline sind weitgehend im Handel erhältlich. Für 2-Amino- bzw. 2-Halogenchinoline seien folgende Literaturstellen genannt: H. Hagen u. R.-D. Kohler, DE 34 12 292; K.M. Johnston et.al., J.C.S. Perkin Trans. I 1972, 1648; K.S.Shorma et.al., Synthesis 1981, 316; Meth-Cohen et.al., Tetrahedron Lett. 1979, 4885; u.a.m.

Die Verbindungen der Formel I, gemäß Anspruch 1 mit der Maßgabe, daß $R^5$ verschieden von Wasserstoff ist, werden z.B. dadurch hergestellt, daß man ein Chinolin der Formel I, in der $R^5$ Wasserstoff ist und X, m, $R^1$, $R^2$, $R^3$, $R^4$, $R^6$, $R^7$ die in Anspruch 1 angegebene Bedeutung haben, mit einem Alkylierungs-, Acylierungs- oder Sulfonylierungsmittel der Formel IV,

$LR^5$ IV

in der

$R^5$ die in Anspruch 1 angegebene Bedeutung außer Wasserstoff hat und L für eine nucleophil verdrängbare Abgangsgruppe steht, in Substanz oder in Gegenwart eines Lösungs- oder Verdünnungsmittels und gegebenenfalls in Gegenwart einer organischen oder anorganischen Base bei Temperaturen zwischen -20 °C und 150 °C umsetzt.

Als nucleophil verdrängbare Gruppen eignen sich bevorzugt Halogenatome wie F, Cl, Br, J, Sulfonate

(Methansulfonat, Benzolsulfonat u.a.) Alkoholate (Ethylat, Benzylat u.a.) und Carboxylate (Acetat u.a.).

Als Basen können die gleichen Verbindungen wie bei der Herstellung von Verbindungen der Formel I ($R^5$ = H) aus Verbindungen der Formeln II und III verwendet werden.

Die Reaktionstemperaturen liegen i.a. zwischen -20° C und 150° C bzw. am Siedepunkt des Lösungsmittels oder Lösungsmittelgemisches.

Bei der Reaktion können ein oder mehrere reaktionsinerte Lösungs- oder Verdünnungsmittel anwesend sein. In Frage kommen beispielsweise aliphatische und aromatische Kohlenwasserstoffe wie Toluol, Xylole, Cyclohexan, Petrolether; halogenierte Kohlenwasserstoffe wie Chlorbenzol, Methylenchlorid, Ethylenchlorid, Chloroform, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Zur Isolierung der neuen Verbindungen wird nach üblichen Methoden vorgegangen. Die anfallenden Produkte können durch Umkristallisieren, Extrahieren oder Chromatographieren gereinigt werden. Folgende Beispiele sollen die Vorgehensweise illustrieren:

Beispiel 1

Zu einer Suspension von 16,9 g (0,1 Mol) 2-Amino-3-cyano-chinolin in 200 ml Tetrahydrofuran (THF) werden bei Raumtemperatur (20° C) portionsweise 12,3 g (0,22 Mol) fein pulverisiertes festes K0H zugegeben. Nach 10 Min. Rühren wird eine Lösung von 27,1 g (0,1 Mol) 6-Trifluormethyl-2,4-dinitrochlorbenzol in 100 ml THF zugetropft. Nach 4 Std. Rühren bei Raumtemperatur wird 1 l Wasser zugegeben, mit Schwefelsäure auf pH ca. 6 gestellt und 3 mal mit je 500 ml Diethylether extrahiert. Die vereinigten Etherextrakte werden mit gesättigter NaCl-Lösung gewaschen, über $MgSO_4$ getrocknet, eingeengt und der Rückstand aus Ethanol umkristallisiert; man erhält 15,3 g (38 %) Verbindung 39, gelbe Kristalle, Schmp. 212° C.

Beispiel 2

Zu einer Lösung von 43,7 g (0,1 Mol) Verbindung 26 in 300 ml THF werden langsam 2,4 g (0,1 Mol) NaH portionsweise zugegeben. Nach 15 Min. werden bei Raumtemperatur 42 g (0,3 Mol) Methyliodid zugetropft. Nach 5 Tagen bei 60° C wird mit 1 l Wasser verdünnt und 3 mal mit je 500 ml Ether extrahiert. Die vereinigten Etherextrakte werden mit ges. NaCl-Lösung gewaschen, über $MgSO_4$ getrocknet, eingeengt und der Rückstand aus Ethanol umkristallisiert; man erhält 35,2 g (78 %) Verbindung 555, gelbe Kristalle, Schmp. 190-194° C.

Entsprechend diesen Herstellungsvorschriften können die in den folgenden Tabellen beschriebenen Verbindungen hergestellt werden.

Tabelle 1

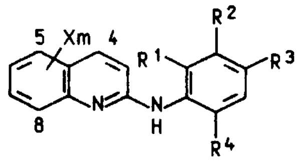

| Nr. | Xm | $R^1$ | $R^2$ | $R^3$ | $R^4$ | Schmp. [°C] |
|---|---|---|---|---|---|---|
| 1 | - | $NO_2$ | H | $CF_3$ | $NO_2$ | |
| 2 | - | $NO_2$ | Cl | $CF_3$ | $NO_2$ | |
| 3 | - | $NO_2$ | H | CN | $NO_2$ | |
| 4 | - | $NO_2$ | Cl | Cl | $NO_2$ | |
| 5 | - | $NO_2$ | H | $NO_2$ | $CF_3$ | |
| 6 | - | $NO_2$ | H | $NO_2$ | Cl | |
| 7 | 6-F | $NO_2$ | H | $CF_3$ | $NO_2$ | |
| 8 | 6-F | $NO_2$ | Cl | $CF_3$ | $NO_2$ | |
| 9 | 6-F | $NO_2$ | H | CN | $NO_2$ | |
| 10 | 6-F | $NO_2$ | Cl | Cl | $NO_2$ | |
| 11 | 6-F | $NO_2$ | H | $NO_2$ | $CF_3$ | |
| 12 | 6-F | $NO_2$ | H | $NO_2$ | Cl | |
| 13 | 6-Cl | $NO_2$ | H | $CF_3$ | $NO_2$ | |
| 14 | 6-Cl | $NO_2$ | Cl | $CF_3$ | $NO_2$ | |
| 15 | 6-Cl | $NO_2$ | H | CN | $NO_2$ | |
| 16 | 6-Cl | $NO_2$ | Cl | Cl | $NO_2$ | |
| 17 | 6-Cl | $NO_2$ | H | $NO_2$ | $CF_3$ | |
| 18 | 6-Cl | $NO_2$ | H | $NO_2$ | Cl | |
| 19 | 6-Br | $NO_2$ | H | $CF_3$ | $NO_2$ | |
| 20 | 6-Br | $NO_2$ | Cl | $CF_3$ | $NO_2$ | |
| 21 | 6-Br | $NO_2$ | H | CN | $NO_2$ | |
| 22 | 6-Br | $NO_2$ | Cl | Cl | $NO_2$ | |
| 23 | 6-Br | $NO_2$ | H | $NO_2$ | $CF_3$ | |
| 24 | 6-Br | $NO_2$ | H | $NO_2$ | Cl | |
| 25 | 3-CN | $NO_2$ | H | $CF_3$ | $NO_2$ | > 220 |
| 26 | 3-CN | $NO_2$ | Cl | $CF_3$ | $NO_2$ | 195 |
| 27 | 3-CN | $NO_2$ | F | $CF_3$ | $NO_2$ | |
| 28 | 3-CN | $NO_2$ | H | CN | $NO_2$ | > 220 |
| 29 | 3-CN | $NO_2$ | Cl | CN | $NO_2$ | |
| 30 | 3-CN | $NO_2$ | F | CN | $NO_2$ | |
| 31 | 3-CN | $NO_2$ | H | Cl | $NO_2$ | |
| 32 | 3-CN | $NO_2$ | Cl | Cl | $NO_2$ | |
| 33 | 3-CN | $NO_2$ | F | Cl | $NO_2$ | |

Tabelle 1 - Forts.

| Nr. | Xm | $R^1$ | $R^2$ | $R^3$ | $R^4$ | Schmp. [°C] |
|---|---|---|---|---|---|---|
| 34 | 3-CN | $NO_2$ | H | $SO_2CH_3$ | $NO_2$ | > 220 |
| 35 | 3-CN | $NO_2$ | H | $SO_2N(CH_3)_2$ | $NO_2$ | |
| 36 | 3-CN | $NO_2$ | H | $CH_3$ | $NO_2$ | > 220 |
| 37 | 3-CN | $NO_2$ | H | $C(CH_3)_3$ | $NO_2$ | 208 |
| 38 | 3-CN | $NO_2$ | H | $NO_2$ | $NO_2$ | |
| 39 | 3-CN | $NO_2$ | H | $NO_2$ | $CF_3$ | 212 |
| 40 | 3-CN | $NO_2$ | H | $NO_2$ | $CH_3$ | > 220 |
| 41 | 3-CN | $NO_2$ | H | $NO_2$ | $CH(CH_3)C_2H_5)$ | |
| 42 | 3-CN | $NO_2$ | H | $NO_2$ | $C(CH_3)_3$ | |
| 43 | 3-CN | $NO_2$ | H | $NO_2$ | Cl | 166-69 |
| 44 | 3-CN | $NO_2$ | H | $NO_2$ | Br | |
| 45 | 3-CN | $NO_2$ | H | $NO_2$ | CN | |
| 46 | 3-CN | $NO_2$ | Cl | $NO_2$ | CN | |
| 47 | 3-CN | $NO_2$ | F | $NO_2$ | CN | |
| 48 | 3-CN | $NO_2$ | Cl | $NO_2$ | $CF_3$ | |
| 49 | 3-CN | $NO_2$ | F | $NO_2$ | $CF_3$ | |
| 50 | 3-CN | H | Cl | $NO_2$ | $NO_2$ | |
| 51 | 3-CN | H | Cl | $CF_3$ | $NO_2$ | > 220 |
| 52 | 3-CN | H | $CF_3$ | Cl | H | |
| 53 | 3-CN | H | $CF_3$ | $NO_2$ | $NO_2$ | > 220 |
| 54 | 3-CN | H | H | $CF_3$ | $NO_2$ | > 220 |
| 55 | 3-CN | H | $NO_2$ | Cl | H | |
| 56 | 3-CN | H | H | $NO_2$ | $CF_3$ | 189-95 |
| 57 | 3-CN | H | H | $NO_2$ | $NO_2$ | |
| 58 | 3-CN | CN | H | $NO_2$ | Cl | |
| 59 | 3-CN | Cl | Cl | Cl | $NO_2$ | |
| 60 | 3-CN, 6-$NO_2$ | $NO_2$ | H | $CF_3$ | $NO_2$ | |
| 61 | 3-CN, 6-$NO_2$ | $NO_2$ | Cl | $CF_3$ | $NO_2$ | |
| 62 | 3-CN, 6-$NO_2$ | $NO_2$ | F | $CF_3$ | $NO_2$ | |
| 63 | 3-CN, 6-$NO_2$ | $NO_2$ | H | CN | $NO_2$ | |
| 64 | 3-CN, 6-$NO_2$ | $NO_2$ | Cl | CN | $NO_2$ | |
| 65 | 3-CN, 6-$NO_2$ | $NO_2$ | F | CN | $NO_2$ | |
| 66 | 3-CN, 6-$NO_2$ | $NO_2$ | H | Cl | $NO_2$ | |
| 67 | 3-CN, 6-$NO_2$ | $NO_2$ | Cl | Cl | $NO_2$ | |
| 68 | 3-CN, 6-$NO_2$ | $NO_2$ | F | Cl | $NO_2$ | |
| 69 | 3-CN, 6-$NO_2$ | $NO_2$ | H | $SO_2CH_3$ | $NO_2$ | |
| 70 | 3-CN, 6-$NO_2$ | $NO_2$ | H | $SO_2N(CH_3)_2$ | $NO_2$ | |
| 71 | 3-CN, 6-$NO_2$ | $NO_2$ | $CH_3$ | H | $NO_2$ | |
| 72 | 3-CN, 6-$NO_2$ | $NO_2$ | H | $C(CH_3)_3$ | $NO_2$ | |
| 73 | 3-CN, 6-$NO_2$ | $NO_2$ | H | $NO_2$ | $NO_2$ | |

Tabelle 1 - Forts.

| Nr. | Xm | $R^1$ | $R^2$ | $R^3$ | $R^4$ | Schmp. [°C] |
|---|---|---|---|---|---|---|
| 74 | 3-CN, 6-$NO_2$ | $NO_2$ | H | $NO_2$ | $CF_3$ | |
| 75 | 3-CN, 6-$NO_2$ | $NO_2$ | H | $NO_2$ | $CH_3$ | |
| 76 | 3-CN, 6-$NO_2$ | $NO_2$ | H | $NO_2$ | $CH(CH_3)(C_2H_5)$ | |
| 77 | 3-CN, 6-$NO_2$ | $NO_2$ | H | $NO_2$ | $C(CH_3)_3$ | |
| 78 | 3-CN, 6-$NO_2$ | $NO_2$ | H | $NO_2$ | Cl | |
| 79 | 3-CN, 6-$NO_2$ | $NO_2$ | H | $NO_2$ | Br | |
| 80 | 3-CN, 6-$NO_2$ | $NO_2$ | H | $NO_2$ | CN | |
| 81 | 3-CN, 6-$NO_2$ | $NO_2$ | Cl | $NO_2$ | CN | |
| 82 | 3-CN, 6-$NO_2$ | $NO_2$ | F | $NO_2$ | CN | |
| 83 | 3-CN, 6-$NO_2$ | $NO_2$ | Cl | $NO_2$ | $CF_3$ | |
| 84 | 3-CN, 6-$NO_2$ | $NO_2$ | F | $NO_2$ | $CF_3$ | |
| 85 | 3-CN, 6-$NO_2$ | H | $CF_3$ | Cl | H | |
| 86 | 3-CN, 6-$NO_2$ | H | $CF_3$ | $NO_2$ | $NO_2$ | |
| 87 | 3-CN, 6-$NO_2$ | H | $NO_2$ | Cl | H | |
| 88 | 3-CN, 6-$NO_2$ | CN | H | $NO_2$ | Cl | |
| 89 | 3-CN, 6-$NO_2$ | Cl | Cl | Cl | $NO_2$ | |
| 90 | 3-CN, 6-Cl | $NO_2$ | H | $CF_3$ | $NO_2$ | |
| 91 | 3-CN, 6-Cl | $NO_2$ | Cl | $CF_3$ | $NO_2$ | |
| 92 | 3-CN, 6-Cl | $NO_2$ | F | $CF_3$ | $NO_2$ | |
| 93 | 3-CN, 6-Cl | $NO_2$ | H | CN | $NO_2$ | |
| 94 | 3-CN, 6-Cl | $NO_2$ | Cl | CN | $NO_2$ | |
| 95 | 3-CN, 6-Cl | $NO_2$ | F | CN | $NO_2$ | |
| 96 | 3-CN, 6-Cl | $NO_2$ | H | Cl | $NO_2$ | |
| 97 | 3-CN, 6-Cl | $NO_2$ | Cl | Cl | $NO_2$ | |
| 98 | 3-CN, 6-Cl | $NO_2$ | H | $SO_2CH_3$ | $NO_2$ | |
| 99 | 3-CN, 6-Cl | $NO_2$ | H | $SO_2N(CH_3)_2$ | $NO_2$ | |
| 100 | 3-CN, 6-Cl | $NO_2$ | H | $NO_2$ | $CF_3$ | |
| 101 | 3-CN, 6-Cl | $NO_2$ | H | $NO_2$ | Cl | |
| 102 | 3-CN, 6-Cl | $NO_2$ | H | $NO_2$ | Br | |
| 103 | 3-CN, 6-Cl | $NO_2$ | H | $NO_2$ | CN | |
| 104 | 3-CN, 6-Cl | $NO_2$ | Cl | $NO_2$ | CN | |
| 105 | 3-CN, 6-Cl | $NO_2$ | Cl | $NO_2$ | $CF_3$ | |
| 106 | 3-CN, 6-Cl | CN | H | $NO_2$ | Cl | |
| 107 | 3-CN, 7-Cl | $NO_2$ | H | $CF_3$ | $NO_2$ | |
| 108 | 3-CN, 7-Cl | $NO_2$ | Cl | $CF_3$ | $NO_2$ | |
| 109 | 3-CN, 7-Cl | $NO_2$ | F | $CF_3$ | $NO_2$ | |
| 110 | 3-CN, 7-Cl | $NO_2$ | H | CN | $NO_2$ | |
| 111 | 3-CN, 7-Cl | $NO_2$ | Cl | CN | $NO_2$ | |
| 112 | 3-CN, 7-Cl | $NO_2$ | H | Cl | $NO_2$ | |
| 113 | 3-CN, 7-Cl | $NO_2$ | Cl | Cl | $NO_2$ | |

Tabelle 1 - Forts.

| Nr. | Xm | $R^1$ | $R^2$ | $R^3$ | $R^4$ | Schmp. [°C] |
|---|---|---|---|---|---|---|
| 114 | 3-CN, 7-Cl | $NO_2$ | H | $SO_2CH_3$ | $NO_2$ | |
| 115 | 3-CN, 7-Cl | $NO_2$ | H | $SO_2N(CH_3)_2$ | $NO_2$ | |
| 116 | 3-CN, 7-Cl | $NO_2$ | H | $NO_2$ | $CF_3$ | |
| 117 | 3-CN, 7-Cl | $NO_2$ | H | $NO_2$ | Cl | |
| 118 | 3-CN, 7-Cl | $NO_2$ | H | $NO_2$ | CN | |
| 119 | 3-CN, 7-Cl | $NO_2$ | Cl | $NO_2$ | CN | |
| 120 | 3-CN, 7-Cl | $NO_2$ | Cl | $NO_2$ | $CF_3$ | |
| 121 | 3-CN, 4-$CH_3$ | $NO_2$ | H | $CF_3$ | $NO_2$ | |
| 122 | 3-CN, 4-$CH_3$ | $NO_2$ | Cl | $CF_3$ | $NO_2$ | |
| 123 | 3-CN, 4-$CH_3$ | $NO_2$ | F | $CF_3$ | $NO_2$ | |
| 124 | 3-CN, 4-$CH_3$ | $NO_2$ | H | CN | $NO_2$ | |
| 125 | 3-CN, 4-$CH_3$ | $NO_2$ | Cl | CN | $NO_2$ | |
| 126 | 3-CN, 4-$CH_3$ | $NO_2$ | H | Cl | $NO_2$ | |
| 127 | 3-CN, 4-$CH_3$ | $NO_2$ | Cl | Cl | $NO_2$ | |
| 128 | 3-CN, 4-$CH_3$ | $NO_2$ | H | $SO_2CH_3$ | $NO_2$ | |
| 129 | 3-CN, 4-$CH_3$ | $NO_2$ | H | $SO_2N(CH_3)_2$ | $NO_2$ | |
| 130 | 3-CN, 4-$CH_3$ | $NO_2$ | H | $NO_2$ | $CF_3$ | |
| 131 | 3-CN, 4-$CH_3$ | $NO_2$ | H | $NO_2$ | Cl | |
| 132 | 3-CN, 4-$CH_3$ | $NO_2$ | H | $NO_2$ | CN | |
| 133 | 3-CN, 4-$CH_3$ | $NO_2$ | Cl | $NO_2$ | CN | |
| 134 | 3-CN, 4-$CH_3$ | $NO_2$ | Cl | $NO_2$ | $CF_3$ | |
| 135 | 3-CN, 6-$CF_3$ | $NO_2$ | H | $CF_3$ | $NO_2$ | |
| 136 | 3-CN, 6-$CF_3$ | $NO_2$ | Cl | $CF_3$ | $NO_2$ | |
| 137 | 3-CN, 6-$CF_3$ | $NO_2$ | F | $CF_3$ | $NO_2$ | |
| 138 | 3-CN, 6-$CF_3$ | $NO_2$ | H | CN | $NO_2$ | |
| 139 | 3-CN, 6-$CF_3$ | $NO_2$ | Cl | CN | $NO_2$ | |
| 140 | 3-CN, 6-$CF_3$ | $NO_2$ | H | Cl | $NO_2$ | |
| 141 | 3-CN, 6-$CF_3$ | $NO_2$ | Cl | Cl | $NO_2$ | |
| 142 | 3-CN, 6-$CF_3$ | $NO_2$ | H | $SO_2CH_3$ | $NO_2$ | |
| 143 | 3-CN, 6-$CF_3$ | $NO_2$ | H | $SO_2N(CH_3)_2$ | $NO_2$ | |
| 144 | 3-CN, 6-$CF_3$ | $NO_2$ | H | $NO_2$ | $CF_3$ | |
| 145 | 3-CN, 6-$CF_3$ | $NO_2$ | H | $NO_2$ | Cl | |
| 146 | 3-CN, 6-$CF_3$ | $NO_2$ | H | $NO_2$ | CN | |
| 147 | 3-CN, 6-$CF_3$ | $NO_2$ | Cl | $NO_2$ | CN | |
| 148 | 3-CN, 6-$CF_3$ | $NO_2$ | Cl | $NO_2$ | $CF_3$ | |
| 149 | 3-CN, 6-$CF_3$ | CN | H | $NO_2$ | Cl | |
| 150 | 3-$CF_3$ | $NO_2$ | H | $CF_3$ | $NO_2$ | |
| 151 | 3-$CF_3$ | $NO_2$ | Cl | $CF_3$ | $NO_2$ | |
| 152 | 3-$CF_3$ | $NO_2$ | F | $CF_3$ | $NO_2$ | |
| 153 | 3-$CF_3$ | $NO_2$ | H | CN | $NO_2$ | |

Tabelle 1 - Forts.

| Nr. | Xm | $R^1$ | $R^2$ | $R^3$ | $R^4$ | Schmp. [°C] |
|---|---|---|---|---|---|---|
| 154 | 3-$CF_3$ | $NO_2$ | Cl | CN | $NO_2$ | |
| 155 | 3-$CF_3$ | $NO_2$ | H | Cl | $NO_2$ | |
| 156 | 3-$CF_3$ | $NO_2$ | Cl | Cl | $NO_2$ | |
| 157 | 3-$CF_3$ | $NO_2$ | H | $SO_2CH_3$ | $NO_2$ | |
| 158 | 3-$CF_3$ | $NO_2$ | H | $SO_2N(CH_3)_2$ | $NO_2$ | |
| 159 | 3-$CF_3$ | $NO_2$ | H | $NO_2$ | $CF_3$ | |
| 160 | 3-$CF_3$ | $NO_2$ | H | $NO_2$ | Cl | |
| 161 | 3-$CF_3$ | $NO_2$ | H | $NO_2$ | CN | |
| 162 | 3-$CF_3$ | $NO_2$ | Cl | $NO_2$ | CN | |
| 163 | 3-$CF_3$ | $NO_2$ | Cl | $NO_2$ | $CF_3$ | |
| 164 | 3-$CF_3$ | CN | H | $NO_2$ | Cl | |
| 165 | 3-$CF_3$ | Cl | Cl | Cl | $NO_2$ | |
| 166 | 3-$CF_3$, 6-$NO_2$ | $NO_2$ | H | $CF_3$ | $NO_2$ | |
| 167 | 3-$CF_3$, 6-$NO_2$ | $NO_2$ | Cl | $CF_3$ | $NO_2$ | |
| 168 | 3-$CF_3$, 6-$NO_2$ | $NO_2$ | F | $CF_3$ | $NO_2$ | |
| 169 | 3-$CF_3$, 6-$NO_2$ | $NO_2$ | H | CN | $NO_2$ | |
| 170 | 3-$CF_3$, 6-$NO_2$ | $NO_2$ | Cl | CN | $NO_2$ | |
| 171 | 3-$CF_3$, 6-$NO_2$ | $NO_2$ | H | Cl | $NO_2$ | |
| 172 | 3-$CF_3$, 6-$NO_2$ | $NO_2$ | Cl | Cl | $NO_2$ | |
| 173 | 3-$CF_3$, 6-$NO_2$ | $NO_2$ | H | $SO_2CH_3$ | $NO_2$ | |
| 174 | 3-$CF_3$, 6-$NO_2$ | $NO_2$ | H | $SO_2N(CH_3)_2$ | $NO_2$ | |
| 175 | 3-$CF_3$, 6-$NO_2$ | $NO_2$ | H | $NO_2$ | $CF_3$ | |
| 176 | 3-$CF_3$, 6-$NO_2$ | $NO_2$ | H | $NO_2$ | Cl | |
| 177 | 3-$CF_3$, 6-$NO_2$ | $NO_2$ | H | $NO_2$ | CN | |
| 178 | 3-$CF_3$, 6-$NO_2$ | $NO_2$ | Cl | $NO_2$ | CN | |
| 179 | 3-$CF_3$, 6-$NO_2$ | $NO_2$ | Cl | $NO_2$ | $CF_3$ | |
| 180 | 3-$CF_3$, 6-$NO_2$ | CN | H | $NO_2$ | Cl | |
| 181 | 3-$CF_3$, 6-Cl | $NO_2$ | H | $CF_3$ | $NO_2$ | |
| 182 | 3-$CF_3$, 6-Cl | $NO_2$ | Cl | $CF_3$ | $NO_2$ | |
| 183 | 3-$CF_3$, 6-Cl | $NO_2$ | F | $CF_3$ | $NO_2$ | |
| 184 | 3-$CF_3$, 6-Cl | $NO_2$ | H | CN | $NO_2$ | |
| 185 | 3-$CF_3$, 6-Cl | $NO_2$ | Cl | CN | $NO_2$ | |
| 186 | 3-$CF_3$, 6-Cl | $NO_2$ | H | Cl | $NO_2$ | |
| 187 | 3-$CF_3$, 6-Cl | $NO_2$ | Cl | Cl | $NO_2$ | |
| 188 | 3-$CF_3$, 6-Cl | $NO_2$ | H | $SO_2CH_3$ | $NO_2$ | |
| 189 | 3-$CF_3$, 6-Cl | $NO_2$ | H | $SO_2N(CH_3)_2$ | $NO_2$ | |
| 190 | 3-$CF_3$, 6-Cl | $NO_2$ | H | $NO_2$ | $CF_3$ | |
| 191 | 3-$CF_3$, 6-Cl | $NO_2$ | H | $NO_2$ | Cl | |
| 192 | 3-$CF_3$, 6-Cl | $NO_2$ | H | $NO_2$ | CN | |
| 193 | 3-$CF_3$, 6-Cl | $NO_2$ | Cl | $NO_2$ | CN | |

Tabelle 1 - Forts.

| Nr. | Xm | $R^1$ | $R^2$ | $R^3$ | $R^4$ | Schmp. [°C] |
|---|---|---|---|---|---|---|
| 194 | 3-$CF_3$, 6-Cl | $NO_2$ | Cl | $NO_2$ | $CF_3$ | |
| 195 | 3-$CF_3$, 6-Cl | CN | H | $NO_2$ | Cl | |
| 196 | 3-$CF_3$, 7-Cl | $NO_2$ | H | $CF_3$ | $NO_2$ | |
| 197 | 3-$CF_3$, 7-Cl | $NO_2$ | Cl | $CF_3$ | $NO_2$ | |
| 198 | 3-$CF_3$, 7-Cl | $NO_2$ | F | $CF_3$ | $NO_2$ | |
| 199 | 3-$CF_3$, 7-Cl | $NO_2$ | H | CN | $NO_2$ | |
| 200 | 3-$CF_3$, 7-Cl | $NO_2$ | Cl | CN | $NO_2$ | |
| 201 | 3-$CF_3$, 7-Cl | $NO_2$ | H | Cl | $NO_2$ | |
| 202 | 3-$CF_3$, 7-Cl | $NO_2$ | Cl | Cl | $NO_2$ | |
| 203 | 3-$CF_3$, 7-Cl | $NO_2$ | H | $SO_2CH_3$ | $NO_2$ | |
| 204 | 3-$CF_3$, 7-Cl | $NO_2$ | H | $SO_2N(CH_3)_2$ | $NO_2$ | |
| 205 | 3-$CF_3$, 7-Cl | $NO_2$ | H | $NO_2$ | $CF_3$ | |
| 206 | 3-$CF_3$, 7-Cl | $NO_2$ | H | $NO_2$ | Cl | |
| 207 | 3-$CF_3$, 7-Cl | $NO_2$ | H | $NO_2$ | CN | |
| 208 | 3-$CF_3$, 7-Cl | $NO_2$ | Cl | $NO_2$ | CN | |
| 209 | 3-$CF_3$, 7-Cl | $NO_2$ | Cl | $NO_2$ | $CF_3$ | |
| 210 | 3-$CF_3$, 7-Cl | CN | H | $NO_2$ | Cl | |
| 211 | 3-$CF_3$, 4-$CH_3$ | $NO_2$ | H | $CF_3$ | $NO_2$ | |
| 212 | 3-$CF_3$, 4-$CH_3$ | $NO_2$ | Cl | $CF_3$ | $NO_2$ | |
| 213 | 3-$CF_3$, 4-$CH_3$ | $NO_2$ | F | $CF_3$ | $NO_2$ | |
| 214 | 3-$CF_3$, 4-$CH_3$ | $NO_2$ | H | CN | $NO_2$ | |
| 215 | 3-$CF_3$, 4-$CH_3$ | $NO_2$ | Cl | CN | $NO_2$ | |
| 216 | 3-$CF_3$, 4-$CH_3$ | $NO_2$ | H | Cl | $NO_2$ | |
| 217 | 3-$CF_3$, 4-$CH_3$ | $NO_2$ | Cl | Cl | $NO_2$ | |
| 218 | 3-$CF_3$, 4-$CH_3$ | $NO_2$ | H | $SO_2CH_3$ | $NO_2$ | |
| 219 | 3-$CF_3$, 4-$CH_3$ | $NO_2$ | H | $SO_2N(CH_3)_2$ | $NO_2$ | |
| 220 | 3-$CF_3$, 4-$CH_3$ | $NO_2$ | H | $NO_2$ | $CF_3$ | |
| 221 | 3-$CF_3$, 4-$CH_3$ | $NO_2$ | H | $NO_2$ | Cl | |
| 222 | 3-$CF_3$, 4-$CH_3$ | $NO_2$ | H | $NO_2$ | CN | |
| 223 | 3-$CF_3$, 4-$CH_3$ | $NO_2$ | Cl | $NO_2$ | CN | |
| 224 | 3-$CF_3$, 4-$CH_3$ | $NO_2$ | Cl | $NO_2$ | $CF_3$ | |
| 225 | 3-$CF_3$, 4-$CH_3$ | CN | H | $NO_2$ | Cl | |
| 226 | 3-$CF_3$, 6-$CH_3$ | $NO_2$ | H | $CF_3$ | $NO_2$ | |
| 227 | 3-$CF_3$, 6-$CH_3$ | $NO_2$ | Cl | $CF_3$ | $NO_2$ | |
| 228 | 3-$CF_3$, 6-$CH_3$ | $NO_2$ | H | CN | $NO_2$ | |
| 229 | 3-$CF_3$, 6-$CH_3$ | $NO_2$ | Cl | Cl | $NO_2$ | |
| 230 | 3-$CF_3$, 6-$CH_3$ | $NO_2$ | H | $SO_2CH_3$ | $NO_2$ | |
| 231 | 3-$CF_3$, 6-$CH_3$ | $NO_2$ | H | $NO_2$ | $CF_3$ | |
| 232 | 3-$CF_3$, 6-$CH_3$ | $NO_2$ | H | $NO_2$ | Cl | |
| 233 | 3-$CF_3$, 6-$CF_3$ | $NO_2$ | H | $CF_3$ | $NO_2$ | |

Tabelle 1 - Forts.

| Nr. | Xm | $R^1$ | $R^2$ | $R^3$ | $R^4$ | Schmp. [°C] |
|---|---|---|---|---|---|---|
| 234 | 3-$CF_3$, 6-$CF_3$ | $NO_2$ | Cl | $CF_3$ | $NO_2$ | |
| 235 | 3-$CF_3$, 6-$CF_3$ | $NO_2$ | F | $CF_3$ | $NO_2$ | |
| 236 | 3-$CF_3$, 6-$CF_3$ | $NO_2$ | H | CN | $NO_2$ | |
| 237 | 3-$CF_3$, 6-$CF_3$ | $NO_2$ | Cl | CN | $NO_2$ | |
| 238 | 3-$CF_3$, 6-$CF_3$ | $NO_2$ | H | Cl | $NO_2$ | |
| 239 | 3-$CF_3$, 6-$CF_3$ | $NO_2$ | Cl | Cl | $NO_2$ | |
| 240 | 3-$CF_3$, 6-$CF_3$ | $NO_2$ | H | $SO_2CH_3$ | $NO_2$ | |
| 241 | 3-$CF_3$, 6-$CF_3$ | $NO_2$ | H | $SO_2N(CH_3)_2$ | $NO_2$ | |
| 242 | 3-$CF_3$, 6-$CF_3$ | $NO_2$ | H | $NO_2$ | $CF_3$ | |
| 243 | 3-$CF_3$, 6-$CF_3$ | $NO_2$ | H | $NO_2$ | Cl | |
| 244 | 3-$CF_3$, 6-$CF_3$ | $NO_2$ | H | $NO_2$ | CN | |
| 245 | 3-$CF_3$, 6-$CF_3$ | $NO_2$ | Cl | $NO_2$ | CN | |
| 246 | 3-$CF_3$, 6-$CF_3$ | $NO_2$ | Cl | $NO_2$ | $CF_3$ | |
| 247 | 3-$CF_3$, 6-$CF_3$ | CN | H | $NO_2$ | Cl | |
| 248 | 3-$CF_3$, 6-$CCl_3$ | $NO_2$ | H | $CF_3$ | $NO_2$ | |
| 249 | 3-$CF_3$, 6-$CCl_3$ | $NO_2$ | Cl | $CF_3$ | $NO_2$ | |
| 250 | 3-$CF_3$, 6-$CCl_3$ | $NO_2$ | H | CN | $NO_2$ | |
| 251 | 3-$CF_3$, 6-$CCl_3$ | $NO_2$ | Cl | Cl | $NO_2$ | |
| 252 | 3-$CF_3$, 6-$CCl_3$ | $NO_2$ | H | $SO_2CH_3$ | $NO_2$ | |
| 253 | 3-$CF_3$, 6-$CCl_3$ | $NO_2$ | H | $NO_2$ | $CF_3$ | |
| 254 | 3-$CF_3$, 6-$CCl_3$ | $NO_2$ | H | $NO_2$ | Cl | |
| 255 | 4-$CF_3$, 6-Cl | $NO_2$ | H | $CF_3$ | $NO_2$ | |
| 256 | 4-$CF_3$, 6-Cl | $NO_2$ | Cl | $CF_3$ | $NO_2$ | |
| 257 | 4-$CF_3$, 6-Cl | $NO_2$ | H | CN | $NO_2$ | |
| 258 | 4-$CF_3$, 6-Cl | $NO_2$ | Cl | Cl | $NO_2$ | |
| 259 | 4-$CF_3$, 6-Cl | $NO_2$ | H | $SO_2CH_3$ | $NO_2$ | |
| 260 | 4-$CF_3$, 6-Cl | $NO_2$ | H | $NO_2$ | $CF_3$ | |
| 261 | 4-$CF_3$, 6-Cl | $NO_2$ | H | $NO_2$ | Cl | |
| 262 | 3-$CHF_2$ | $NO_2$ | H | $CF_3$ | $NO_2$ | |
| 263 | 3-$CHF_2$ | $NO_2$ | Cl | $CF_3$ | $NO_2$ | |
| 264 | 3-$CHF_2$ | $NO_2$ | H | CN | $NO_2$ | |
| 265 | 3-$CHF_2$ | $NO_2$ | Cl | Cl | $NO_2$ | |
| 266 | 3-$CHF_2$ | $NO_2$ | H | $SO_2CH_3$ | $NO_2$ | |
| 267 | 3-$CHF_2$ | $NO_2$ | H | $NO_2$ | $CF_3$ | |
| 268 | 3-$CHF_2$ | $NO_2$ | H | $NO_2$ | Cl | |
| 269 | 3-$CHF_2$ | CN | H | $NO_2$ | Cl | |
| 270 | 3-$CHF_2$, 6-$NO_2$ | $NO_2$ | H | $CF_3$ | $NO_2$ | |
| 271 | 3-$CHF_2$, 6-$NO_2$ | $NO_2$ | Cl | $CF_3$ | $NO_2$ | |
| 272 | 3-$CHF_2$, 6-$NO_2$ | $NO_2$ | H | CN | $NO_2$ | |
| 273 | 3-$CHF_2$, 6-$NO_2$ | $NO_2$ | Cl | Cl | $NO_2$ | |

Tabelle 1 - Forts.

| Nr. | Xm | $R^1$ | $R^2$ | $R^3$ | $R^4$ | Schmp. [°C] |
|---|---|---|---|---|---|---|
| 274 | 3-$CHF_2$, 6-$NO_2$ | $NO_2$ | H | $SO_2CH_3$ | $NO_2$ | |
| 275 | 3-$CHF_2$, 6-$NO_2$ | $NO_2$ | H | $NO_2$ | $CF_3$ | |
| 276 | 3-$CHF_2$, 6-$NO_2$ | $NO_2$ | H | $NO_2$ | Cl | |
| 277 | 3-$CHF_2$, 6-$CH_3$ | $NO_2$ | H | $CF_3$ | $NO_2$ | |
| 278 | 3-$CHF_2$, 6-$CH_3$ | $NO_2$ | Cl | $CF_3$ | $NO_2$ | |
| 279 | 3-$CHF_2$, 6-$CH_3$ | $NO_2$ | H | CN | $NO_2$ | |
| 280 | 3-$CHF_2$, 6-$CH_3$ | $NO_2$ | Cl | Cl | $NO_2$ | |
| 281 | 3-$CHF_2$, 6-$CH_3$ | $NO_2$ | H | $SO_2CH_3$ | $NO_2$ | |
| 282 | 3-$CHF_2$, 6-$CH_3$ | $NO_2$ | H | $NO_2$ | $CF_3$ | |
| 283 | 3-$CHF_2$, 6-$CH_3$ | $NO_2$ | H | $NO_2$ | Cl | |
| 284 | 3-$CHF_2$, 6-$CH_3$ | CN | H | $NO_2$ | Cl | |
| 285 | 3-$CHF_2$, 6-$CF_3$ | $NO_2$ | H | $CF_3$ | $NO_2$ | |
| 286 | 3-$CHF_2$, 6-$CF_3$ | $NO_2$ | Cl | $CF_3$ | $NO_2$ | |
| 287 | 3-$CHF_2$, 6-$CF_3$ | $NO_2$ | H | CN | $NO_2$ | |
| 288 | 3-$CHF_2$, 6-$CF_3$ | $NO_2$ | Cl | Cl | $NO_2$ | |
| 289 | 3-$CHF_2$, 6-$CF_3$ | $NO_2$ | H | $SO_2CH_3$ | $NO_2$ | |
| 290 | 3-$CHF_2$, 6-$CF_3$ | $NO_2$ | H | $NO_2$ | $CF_3$ | |
| 291 | 3-$CHF_2$, 6-$CF_3$ | $NO_2$ | H | $NO_2$ | Cl | |
| 292 | 3-$CHF_2$, 6-$CCl_3$ | $NO_2$ | H | $CF_3$ | $NO_2$ | |
| 293 | 3-$CHF_2$, 6-$CCl_3$ | $NO_2$ | Cl | $CF_3$ | $NO_2$ | |
| 294 | 3-$CHF_2$, 6-$CCl_3$ | $NO_2$ | H | CN | $NO_2$ | |
| 295 | 3-$CHF_2$, 6-$CCl_3$ | $NO_2$ | Cl | Cl | $NO_2$ | |
| 296 | 3-$CHF_2$, 6-$CCl_3$ | $NO_2$ | H | $SO_2CH_3$ | $NO_2$ | |
| 297 | 3-$CHF_2$, 6-$CCl_3$ | $NO_2$ | H | $NO_2$ | $CF_3$ | |
| 298 | 3-$CHF_2$, 6-$CCl_3$ | $NO_2$ | H | $NO_2$ | Cl | |
| 299 | 3-Cl | $NO_2$ | H | $CF_3$ | $NO_2$ | |
| 300 | 3-Cl | $NO_2$ | Cl | $CF_3$ | $NO_2$ | |
| 301 | 3-Cl | $NO_2$ | F | $CF_3$ | $NO_2$ | |
| 302 | 3-Cl | $NO_2$ | H | CN | $NO_2$ | |
| 303 | 3-Cl | $NO_2$ | Cl | CN | $NO_2$ | |
| 304 | 3-Cl | $NO_2$ | H | Cl | $NO_2$ | |
| 305 | 3-Cl | $NO_2$ | Cl | Cl | $NO_2$ | |
| 306 | 3-Cl | $NO_2$ | H | $SO_2CH_3$ | $NO_2$ | |
| 307 | 3-Cl | $NO_2$ | H | $SO_2N(CH_3)_2$ | $NO_2$ | |
| 308 | 3-Cl | $NO_2$ | H | $NO_2$ | $CF_3$ | |
| 309 | 3-Cl | $NO_2$ | H | $NO_2$ | Cl | |
| 310 | 3-Cl | $NO_2$ | H | $NO_2$ | CN | |
| 311 | 3-Cl | $NO_2$ | Cl | $NO_2$ | CN | |
| 312 | 3-Cl | $NO_2$ | Cl | $NO_2$ | $CF_3$ | |
| 313 | 3-Cl | CN | H | $NO_2$ | Cl | |

Tabelle 1 - Forts.

| Nr. | Xm | $R^1$ | $R^2$ | $R^3$ | $R^4$ | Schmp. [°C] |
|---|---|---|---|---|---|---|
| 314 | 3-Cl | Cl | Cl | Cl | $NO_2$ | |
| 315 | 3-Cl, 4-$CH_3$ | $NO_2$ | H | $CF_3$ | $NO_2$ | |
| 316 | 3-Cl, 4-$CH_3$ | $NO_2$ | Cl | $CF_3$ | $NO_2$ | |
| 317 | 3-Cl, 4-$CH_3$ | $NO_2$ | H | CN | $NO_2$ | |
| 318 | 3-Cl, 4-$CH_3$ | $NO_2$ | Cl | Cl | $NO_2$ | |
| 319 | 3-Cl, 4-$CH_3$ | $NO_2$ | H | $SO_2CH_3$ | $NO_2$ | |
| 320 | 3-Cl, 4-$CH_3$ | $NO_2$ | H | $NO_2$ | $CF_3$ | |
| 321 | 3-Cl, 4-$CH_3$ | $NO_2$ | H | $NO_2$ | Cl | |
| 322 | 3-Cl, 4-$CH_3$ | CN | H | $NO_2$ | Cl | |
| 323 | 3,6-$Cl_2$ | $NO_2$ | H | $CF_3$ | $NO_2$ | |
| 324 | 3,6-$Cl_2$ | $NO_2$ | Cl | $CF_3$ | $NO_2$ | |
| 325 | 3,6-$Cl_2$ | $NO_2$ | F | $CF_3$ | $NO_2$ | |
| 326 | 3,6-$Cl_2$ | $NO_2$ | H | CN | $NO_2$ | |
| 327 | 3,6-$Cl_2$ | $NO_2$ | Cl | CN | $NO_2$ | |
| 328 | 3,6-$Cl_2$ | $NO_2$ | H | Cl | $NO_2$ | |
| 329 | 3,6-$Cl_2$ | $NO_2$ | Cl | Cl | $NO_2$ | |
| 330 | 3,6-$Cl_2$ | $NO_2$ | H | $SO_2CH_3$ | $NO_2$ | |
| 331 | 3,6-$Cl_2$ | $NO_2$ | H | $SO_2N(CH_3)_2$ | $NO_2$ | |
| 332 | 3,6-$Cl_2$ | $NO_2$ | H | $NO_2$ | $CF_3$ | |
| 333 | 3,6-$Cl_2$ | $NO_2$ | H | $NO_2$ | Cl | |
| 334 | 3,6-$Cl_2$ | $NO_2$ | H | $NO_2$ | CN | |
| 335 | 3,6-$Cl_2$ | $NO_2$ | Cl | $NO_2$ | CN | |
| 336 | 3,6-$Cl_2$ | $NO_2$ | Cl | $NO_2$ | $CF_3$ | |
| 337 | 3,6-$Cl_2$ | CN | H | $NO_2$ | Cl | |
| 338 | 3,6-$Br_2$ | $NO_2$ | H | $CF_3$ | $NO_2$ | |
| 339 | 3,6-$Br_2$ | $NO_2$ | Cl | $CF_3$ | $NO_2$ | |
| 340 | 3,6-$Br_2$ | $NO_2$ | H | CN | $NO_2$ | |
| 341 | 3,6-$Br_2$ | $NO_2$ | Cl | Cl | $NO_2$ | |
| 342 | 3,6-$Br_2$ | $NO_2$ | H | $SO_2CH_3$ | $NO_2$ | |
| 343 | 3,6-$Br_2$ | $NO_2$ | H | $NO_2$ | $CF_3$ | |
| 344 | 3,6-$Br_2$ | $NO_2$ | H | $NO_2$ | Cl | |
| 345 | 3-Cl, 6-Br | $NO_2$ | H | $CF_3$ | $NO_2$ | |
| 346 | 3-Cl, 6-Br | $NO_2$ | Cl | $CF_3$ | $NO_2$ | |
| 347 | 3-Cl, 6-Br | $NO_2$ | H | CN | $NO_2$ | |
| 348 | 3-Cl, 6-Br | $NO_2$ | Cl | Cl | $NO_2$ | |
| 349 | 3-Cl, 6-Br | $NO_2$ | H | $SO_2CH_3$ | $NO_2$ | |
| 350 | 3-Cl, 6-Br | $NO_2$ | H | $NO_2$ | $CF_3$ | |
| 351 | 3-Cl, 6-Br | $NO_2$ | H | $NO_2$ | Cl | |
| 352 | 3-Br, 6-Cl | $NO_2$ | H | $CF_3$ | $NO_2$ | |
| 353 | 3-Br, 6-Cl | $NO_2$ | Cl | $CF_3$ | $NO_2$ | |

Tabelle 1 - Forts.

| Nr. | Xm | $R^1$ | $R^2$ | $R^3$ | $R^4$ | Schmp. [°C] |
|---|---|---|---|---|---|---|
| 354 | 3-Br, 6-Cl | $NO_2$ | H | CN | $NO_2$ | |
| 355 | 3-Br, 6-Cl | $NO_2$ | Cl | Cl | $NO_2$ | |
| 356 | 3-Br, 6-Cl | $NO_2$ | H | $SO_2CH_3$ | $NO_2$ | |
| 357 | 3-Br, 6-Cl | $NO_2$ | H | $NO_2$ | $CF_3$ | |
| 358 | 3-Br, 6-Cl | $NO_2$ | H | $NO_2$ | Cl | |
| 359 | 3-Br | $NO_2$ | H | $CF_3$ | $NO_2$ | |
| 360 | 3-Br | $NO_2$ | Cl | $CF_3$ | $NO_2$ | |
| 361 | 3-Br | $NO_2$ | H | CN | $NO_2$ | |
| 362 | 3-Br | $NO_2$ | Cl | Cl | $NO_2$ | |
| 363 | 3-Br | $NO_2$ | H | $SO_2CH_3$ | $NO_2$ | |
| 364 | 3-Br | $NO_2$ | H | $NO_2$ | $CF_3$ | |
| 365 | 3-Br | $NO_2$ | H | $NO_2$ | Cl | |
| 366 | 3-Cl, 6-$NO_2$ | $NO_2$ | H | $CF_3$ | $NO_2$ | |
| 367 | 3-Cl, 6-$NO_2$ | $NO_2$ | Cl | $CF_3$ | $NO_2$ | |
| 368 | 3-Cl, 6-$NO_2$ | $NO_2$ | F | $CF_3$ | $NO_2$ | |
| 369 | 3-Cl, 6-$NO_2$ | $NO_2$ | H | CN | $NO_2$ | |
| 370 | 3-Cl, 6-$NO_2$ | $NO_2$ | Cl | CN | $NO_2$ | |
| 371 | 3-Cl, 6-$NO_2$ | $NO_2$ | H | Cl | $NO_2$ | |
| 372 | 3-Cl, 6-$NO_2$ | $NO_2$ | Cl | Cl | $NO_2$ | |
| 373 | 3-Cl, 6-$NO_2$ | $NO_2$ | H | $SO_2CH_3$ | $NO_2$ | |
| 374 | 3-Cl, 6-$NO_2$ | $NO_2$ | H | $SO_2N(CH_3)_2$ | $NO_2$ | |
| 375 | 3-Cl, 6-$NO_2$ | $NO_2$ | H | $NO_2$ | $CF_3$ | |
| 376 | 3-Cl, 6-$NO_2$ | $NO_2$ | H | $NO_2$ | Cl | |
| 377 | 3-Cl, 6-$NO_2$ | $NO_2$ | H | $NO_2$ | CN | |
| 378 | 3-Cl, 6-$NO_2$ | $NO_2$ | Cl | $NO_2$ | CN | |
| 379 | 3-Cl, 6-$NO_2$ | $NO_2$ | Cl | $NO_2$ | $CF_3$ | |
| 380 | 3-Cl, 6-$NO_2$ | CN | H | $NO_2$ | Cl | |
| 381 | 3-Br, 6-$NO_2$ | $NO_2$ | H | $CF_3$ | $NO_2$ | |
| 382 | 3-Br, 6-$NO_2$ | $NO_2$ | Cl | $CF_3$ | $NO_2$ | |
| 383 | 3-Br, 6-$NO_2$ | $NO_2$ | H | CN | $NO_2$ | |
| 384 | 3-Br, 6-$NO_2$ | $NO_2$ | Cl | Cl | $NO_2$ | |
| 385 | 3-Br, 6-$NO_2$ | $NO_2$ | H | $SO_2CH_3$ | $NO_2$ | |
| 386 | 3-Br, 6-$NO_2$ | $NO_2$ | H | $NO_2$ | $CF_3$ | |
| 387 | 3-Br, 6-$NO_2$ | $NO_2$ | H | $NO_2$ | Cl | |
| 388 | 3-Br, 6-$NO_2$ | CN | H | $NO_2$ | Cl | |
| 389 | 3-Cl, 6-$CF_3$ | $NO_2$ | H | $CF_3$ | $NO_2$ | |
| 390 | 3-Cl, 6-$CF_3$ | $NO_2$ | Cl | $CF_3$ | $NO_2$ | |
| 391 | 3-Cl, 6-$CF_3$ | $NO_2$ | F | $CF_3$ | $NO_2$ | |
| 392 | 3-Cl, 6-$CF_3$ | $NO_2$ | H | CN | $NO_2$ | |
| 393 | 3-Cl, 6-$CF_3$ | $NO_2$ | Cl | CN | $NO_2$ | |

Tabelle 1 - Forts.

| Nr. | Xm | $R^1$ | $R^2$ | $R^3$ | $R^4$ | Schmp. [°C] |
|---|---|---|---|---|---|---|
| 394 | 3-Cl, 6-$CF_3$ | $NO_2$ | H | Cl | $NO_2$ | |
| 395 | 3-Cl, 6-$CF_3$ | $NO_2$ | Cl | Cl | $NO_2$ | |
| 396 | 3-Cl, 6-$CF_3$ | $NO_2$ | H | $SO_2CH_3$ | $NO_2$ | |
| 397 | 3-Cl, 6-$CF_3$ | $NO_2$ | H | $SO_2N(CH_3)_2$ | $NO_2$ | |
| 398 | 3-Cl, 6-$CF_3$ | $NO_2$ | H | $NO_2$ | $CF_3$ | |
| 399 | 3-Cl, 6-$CF_3$ | $NO_2$ | H | $NO_2$ | Cl | |
| 400 | 3-Cl, 6-$CF_3$ | $NO_2$ | H | $NO_2$ | CN | |
| 401 | 3-Cl, 6-$CF_3$ | $NO_2$ | Cl | $NO_2$ | CN | |
| 402 | 3-Cl, 6-$CF_3$ | $NO_2$ | Cl | $NO_2$ | $CF_3$ | |
| 403 | 3-Cl, 6-$CF_3$ | CN | H | $NO_2$ | Cl | |
| 404 | 3-Cl, 6-$CF_3$ | Cl | Cl | Cl | $NO_2$ | |
| 405 | 3-Br, 6-$CF_3$ | $NO_2$ | H | $CF_3$ | $NO_2$ | |
| 406 | 3-Br, 6-$CF_3$ | $NO_2$ | Cl | $CF_3$ | $NO_2$ | |
| 407 | 3-Br, 6-$CF_3$ | $NO_2$ | H | CN | $NO_2$ | |
| 408 | 3-Br, 6-$CF_3$ | $NO_2$ | Cl | Cl | $NO_2$ | |
| 409 | 3-Br, 6-$CF_3$ | $NO_2$ | H | $SO_2CH_3$ | $NO_2$ | |
| 410 | 3-Br, 6-$CF_3$ | $NO_2$ | H | $NO_2$ | $CF_3$ | |
| 411 | 3-Br, 6-$CF_3$ | $NO_2$ | H | $NO_2$ | Cl | |
| 412 | 3-Cl, 4 $CH_3$ | $NO_2$ | H | $CF_3$ | $NO_2$ | |
| 413 | 3-Cl, 4-$CH_3$ | $NO_2$ | Cl | $CF_3$ | $NO_2$ | |
| 414 | 3-Cl, 4-$CH_3$ | $NO_2$ | H | CN | $NO_2$ | |
| 415 | 3-Cl, 4-$CH_3$ | $NO_2$ | Cl | Cl | $NO_2$ | |
| 416 | 3-Cl, 4-$CH_3$ | $NO_2$ | H | $SO_2CH_3$ | $NO_2$ | |
| 417 | 3-Cl, 4-$CH_3$ | $NO_2$ | H | $NO_2$ | $CF_3$ | |
| 418 | 3-Cl, 4-$CH_3$ | $NO_2$ | H | $NO_2$ | Cl | |
| 419 | 3-Cl, 4-$CH_3$, 6-F | $NO_2$ | H | $CF_3$ | $NO_2$ | |
| 420 | 3-Cl, 4-$CH_3$, 6-F | $NO_2$ | Cl | $CF_3$ | $NO_2$ | |
| 421 | 3-Cl, 4-$CH_3$, 6-F | $NO_2$ | F | $CF_3$ | $NO_2$ | |
| 422 | 3-Cl, 4-$CH_3$, 6-F | $NO_2$ | H | CN | $NO_2$ | |
| 423 | 3-Cl, 4-$CH_3$, 6-F | $NO_2$ | Cl | CN | $NO_2$ | |
| 424 | 3-Cl, 4-$CH_3$, 6-F | $NO_2$ | H | Cl | $NO_2$ | |
| 425 | 3-Cl, 4-$CH_3$, 6-F | $NO_2$ | Cl | Cl | $NO_2$ | |
| 426 | 3-Cl, 4-$CH_3$, 6-F | $NO_2$ | H | $SO_2CH_3$ | $NO_2$ | |
| 427 | 3-Cl, 4-$CH_3$, 6-F | $NO_2$ | H | $SO_2N(CH_3)_2$ | $NO_2$ | |
| 428 | 3-Cl, 4-$CH_3$, 6-F | $NO_2$ | H | $NO_2$ | $CF_3$ | |
| 429 | 3-Cl, 4-$CH_3$, 6-F | $NO_2$ | H | $NO_2$ | Cl | |
| 430 | 3-Cl, 4-$CH_3$, 6-F | $NO_2$ | H | $NO_2$ | CN | |
| 431 | 3-Cl, 4-$CH_3$, 6-F | $HO_2$ | Cl | $NO_2$ | CN | |
| 432 | 3-Cl, 4-$CH_3$, 6-F | $NO_2$ | Cl | $NO_2$ | $CF_3$ | |
| 433 | 3-Cl, 4-$CH_3$, 6-F | CN | H | $NO_2$ | Cl | |

Tabelle 1 - Forts.

| Nr. | Xm | $R^1$ | $R^2$ | $R^3$ | $R^4$ | Schmp. [°C] |
|---|---|---|---|---|---|---|
| 434 | 3-Cl, 4-$CH_3$, 6-F | Cl | Cl | Cl | $NO_2$ | |
| 435 | 3-Cl, 4-$CH_3$, 7-F | $NO_2$ | H | $CF_3$ | $NO_2$ | |
| 436 | 3-Cl, 4-$CH_3$, 7-F | $NO_2$ | Cl | $CF_3$ | $NO_2$ | |
| 437 | 3-Cl, 4-$CH_3$, 7-F | $NO_2$ | H | CN | $NO_2$ | |
| 438 | 3-Cl, 4-$CH_3$, 7-F | $NO_2$ | Cl | Cl | $NO_2$ | |
| 439 | 3-Cl, 4-$CH_3$, 7-F | $NO_2$ | H | $SO_2CH_3$ | $NO_2$ | |
| 440 | 3-Cl, 4-$CH_3$, 7-F | $NO_2$ | H | $NO_2$ | $CF_3$ | |
| 441 | 3-Cl, 4-$CH_3$, 7-F | $NO_2$ | H | $NO_2$ | Cl | |
| 442 | 3-Cl, 4-$CH_3$, 7-F | CN | H | $NO_2$ | Cl | |
| 443 | 3,6-$Cl_2$, 4-$CH_3$ | $NO_2$ | H | $CF_3$ | $NO_2$ | |
| 444 | 3,6-$Cl_2$, 4-$CH_3$ | $NO_2$ | Cl | $CF_3$ | $NO_2$ | |
| 445 | 3,6-$Cl_2$, 4-$CH_3$ | $NO_2$ | H | CN | $NO_2$ | |
| 446 | 3,6-$Cl_2$, 4-$CH_3$ | $NO_2$ | Cl | Cl | $NO_2$ | |
| 447 | 3,6-$Cl_2$, 4-$CH_3$ | $NO_2$ | H | $SO_2CH_3$ | $NO_2$ | |
| 448 | 3,6-$Cl_2$, 4-$CH_3$ | $NO_2$ | H | $NO_2$ | $CF_3$ | |
| 449 | 3,6-$Cl_2$, 4-$CH_3$ | $NO_2$ | H | $NO_2$ | Cl | |
| 450 | 3,6-$Cl_2$, 4-$CH_3$ | CN | H | $NO_2$ | Cl | |
| 451 | 3-Cl, 4-$CH_3$, 6-$CF_3$ | $NO_2$ | H | $CF_3$ | $NO_2$ | |
| 452 | 3-Cl, 4-$CH_3$ 6-$CF_3$ | $NO_2$ | Cl | $CF_3$ | $NO_2$ | |
| 453 | 3-Cl, 4-$CH_3$, 6-$CF_3$ | $NO_2$ | H | CN | $NO_2$ | |
| 454 | 3-Cl, 4-$CH_3$, 6-$CF_3$ | $NO_2$ | Cl | Cl | $NO_2$ | |
| 455 | 3-Cl, 4-$CH_3$, 6-$CF_3$ | $NO_2$ | H | $SO_2CH_3$ | $NO_2$ | |
| 456 | 3-Cl, 4-$CH_3$, 6-$CF_3$ | $NO_2$ | H | $NO_2$ | $CF_3$ | |
| 457 | 3-Cl, 4-$CH_3$, 6-$CF_3$ | $NO_2$ | H | $NO_2$ | Cl | |
| 458 | 3-Cl, 4-$CH_3$, 6-$CF_3$ | CN | H | $NO_2$ | Cl | |
| 459 | 3-$NO_2$ | $NO_2$ | H | $CF_3$ | $NO_2$ | |
| 460 | 3-$NO_2$ | $NO_2$ | Cl | $CF_3$ | $NO_2$ | |
| 461 | 3-$NO_2$ | $NO_2$ | H | CN | $NO_2$ | |
| 462 | 3-$NO_2$ | $NO_2$ | Cl | Cl | $NO_2$ | |
| 463 | 3-$NO_2$ | $NO_2$ | H | $SO_2CH_3$ | $NO_2$ | |
| 464 | 3-$NO_2$ | $NO_2$ | H | $NO_2$ | $CF_3$ | |
| 465 | 3-$NO_2$ | $NO_2$ | H | $NO_2$ | Cl | |
| 466 | 3-$NO_2$ | CN | H | $NO_2$ | Cl | |
| 467 | 3-$NO_2$, 6-Cl | $NO_2$ | H | $CF_3$ | $NO_2$ | |
| 468 | 3-$NO_2$, 6-Cl | $NO_2$ | Cl | $CF_3$ | $NO_2$ | |
| 469 | 3-$NO_2$, 6-Cl | $NO_2$ | F | $CF_3$ | $NO_2$ | |
| 470 | 3-$NO_2$, 6-Cl | $NO_2$ | H | CN | $NO_2$ | |
| 471 | 3-$NO_2$, 6-Cl | $NO_2$ | Cl | CN | $NO_2$ | |
| 472 | 3-$NO_2$, 6-Cl | $NO_2$ | H | Cl | $NO_2$ | |
| 473 | 3-$NO_2$, 6-Cl | $NO_2$ | Cl | Cl | $NO_2$ | |

Tabelle 1 - Forts.

| Nr. | Xm | $R^1$ | $R^2$ | $R^3$ | $R^4$ | Schmp. [°C] |
|---|---|---|---|---|---|---|
| 474 | 3-$NO_2$, 6-Cl | $NO_2$ | H | $SO_2CH_3$ | $NO_2$ | |
| 475 | 3-$NO_2$, 6-Cl | $NO_2$ | H | $SO_2N(CH_3)_2$ | $NO_2$ | |
| 476 | 3-$NO_2$, 6-Cl | $NO_2$ | H | $NO_2$ | $CF_3$ | |
| 477 | 3-$NO_2$, 6-Cl | $NO_2$ | H | $NO_2$ | Cl | |
| 478 | 3-$NO_2$, 6-Cl | $NO_2$ | H | $NO_2$ | CN | |
| 479 | 3-$NO_2$, 6-Cl | $HO_2$ | Cl | $NO_2$ | CN | |
| 480 | 3-$NO_2$, 6-Cl | $NO_2$ | Cl | $NO_2$ | $CF_3$ | |
| 481 | 3-$NO_2$, 6-Cl | CN | H | $NO_2$ | Cl | |
| 482 | 3-$NO_2$, 6-Cl | Cl | Cl | Cl | $NO_2$ | |
| 483 | 3-$NO_2$, 6-$CF_3$ | $NO_2$ | H | $CF_3$ | $NO_2$ | |
| 484 | 3-$NO_2$, 6-$CF_3$ | $NO_2$ | Cl | $CF_3$ | $NO_2$ | |
| 485 | 3-$NO_2$, 6-$CF_3$ | $NO_2$ | H | CN | $NO_2$ | |
| 486 | 3-$NO_2$, 6-$CF_3$ | $NO_2$ | Cl | Cl | $NO_2$ | |
| 487 | 3-$NO_2$, 6-$CF_3$ | $NO_2$ | H | $SO_2CH_3$ | $NO_2$ | |
| 488 | 3-$NO_2$, 6-$CF_3$ | $NO_2$ | H | $NO_2$ | $CF_3$ | |
| 489 | 3-$NO_2$, 6-$CF_3$ | $NO_2$ | H | $NO_2$ | Cl | |
| 490 | 3-$NO_2$, 6-$CF_3$ | CN | H | $NO_2$ | Cl | |
| 491 | 3,6-$(NO_2)_2$ | $NO_2$ | H | $CF_3$ | $NO_2$ | |
| 492 | 3,6-$(NO_2)_2$ | $NO_2$ | Cl | $CF_3$ | $NO_2$ | |
| 493 | 3,6-$(NO_2)_2$ | $NO_2$ | H | CN | $NO_2$ | |
| 494 | 3,6-$(NO_2)_2$ | $NO_2$ | Cl | Cl | $NO_2$ | |
| 495 | 3,6-$(NO_2)_2$ | $NO_2$ | H | $SO_2CH_3$ | $NO_2$ | |
| 496 | 3,6-$(NO_2)_2$ | $NO_2$ | H | $NO_2$ | $CF_3$ | |
| 497 | 3,6-$(NO_2)_2$ | $NO_2$ | H | $NO_2$ | Cl | |
| 498 | 3-$NO_2$, 4-$CH_3$ | $NO_2$ | H | $CF_3$ | $NO_2$ | |
| 499 | 3-$NO_2$ 4-$CH_3$ | $NO_2$ | Cl | $CF_3$ | $NO_2$ | |
| 500 | 3-$NO_2$, 4-$CH_3$ | $NO_2$ | H | CN | $NO_2$ | |
| 501 | 3-$NO_2$, 4-$CH_3$ | $NO_2$ | Cl | Cl | $NO_2$ | |
| 502 | 3-$NO_2$, 4-$CH_3$ | $NO_2$ | H | $SO_2CH_3$ | $NO_2$ | |
| 503 | 3-$NO_2$, 4-$CH_3$ | $NO_2$ | H | $NO_2$ | $CF_3$ | |
| 504 | 3-$NO_2$, 4-$CH_3$ | $NO_2$ | H | $NO_2$ | Cl | |
| 505 | 3-$SO_2CH_3$ | $NO_2$ | H | $CF_3$ | $NO_2$ | |
| 506 | 3-$SO_2CH_3$ | $NO_2$ | Cl | $CF_3$ | $NO_2$ | |
| 507 | 3-$SO_2CH_3$ | $NO_2$ | H | CN | $NO_2$ | |
| 508 | 3-$SO_2CH_3$ | $NO_2$ | Cl | Cl | $NO_2$ | |
| 509 | 3-$SO_2CH_3$ | $NO_2$ | H | $SO_2CH_3$ | $NO_2$ | |
| 510 | 3-$SO_2CH_3$ | $NO_2$ | H | $NO_2$ | $CF_3$ | |
| 511 | 3-$SO_2CH_3$ | $NO_2$ | H | $NO_2$ | Cl | |
| 512 | 3-$SO_2CH_3$ | CN | H | $NO_2$ | Cl | |
| 513 | 3-$SO_2CH_3$, 4-$CH_3$ | $NO_2$ | H | $CF_3$ | $NO_2$ | |

Tabelle 1 - Forts.

| Nr. | Xm | $R^1$ | $R^2$ | $R^3$ | $R^4$ | Schmp. [°C] |
|---|---|---|---|---|---|---|
| 514 | 3-$SO_2CH_3$, 4-$CH_3$ | $NO_2$ | Cl | $CF_3$ | $NO_2$ | |
| 515 | 3-$SO_2CH_3$, 4-$CH_3$ | $NO_2$ | H | CN | $NO_2$ | |
| 516 | 3-$SO_2CH_3$, 4-$CH_3$ | $NO_2$ | Cl | Cl | $NO_2$ | |
| 517 | 3-$SO_2CH_3$, 4-$CH_3$ | $NO_2$ | H | $SO_2CH_3$ | $NO_2$ | |
| 518 | 3-$SO_2CH_3$, 4-$CH_3$ | $NO_2$ | H | $NO_2$ | $CF_3$ | |
| 519 | 3-$SO_2CH_3$, 4-$CH_3$ | $NO_2$ | H | $NO_2$ | Cl | |
| 520 | 3-$SO_2CH_3$, 4-$CH_3$ | CN | H | $NO_2$ | Cl | |
| 521 | 3-$SO_2CH_3$, 6-Cl | $NO_2$ | H | $CF_3$ | $NO_2$ | |
| 522 | 3-$SO_2CH_3$, 6-Cl | $NO_2$ | Cl | $CF_3$ | $NO_2$ | |
| 523 | 3-$SO_2CH_3$, 6-Cl | $NO_2$ | H | CN | $NO_2$ | |
| 524 | 3-$SO_2CH_3$, 6-Cl | $NO_2$ | Cl | Cl | $NO_2$ | |
| 525 | 3-$SO_2CH_3$, 6-Cl | $NO_2$ | H | $SO_2CH_3$ | $NO_2$ | |
| 526 | 3-$SO_2CH_3$, 6-Cl | $NO_2$ | H | $NO_2$ | $CF_3$ | |
| 527 | 3-$SO_2CH_3$, 6-Cl | $NO_2$ | H | $NO_2$ | Cl | |
| 528 | 3-$SO_2CH_3$, 6-Cl | CN | H | $NO_2$ | Cl | |
| 529 | 3-$SO_2CH_3$, 6-$CF_3$ | $NO_2$ | H | $CF_3$ | $NO_2$ | |
| 530 | 3-$SO_2CH_3$, 6-$CF_3$ | $NO_2$ | Cl | $CF_3$ | $NO_2$ | |
| 531 | 3-$SO_2CH_3$, 6-$CF_3$ | $NO_2$ | H | CN | $NO_2$ | |
| 532 | 3-$SO_2CH_3$, 6-$CF_3$ | $NO_2$ | Cl | Cl | $NO_2$ | |
| 533 | 3-$SO_2CH_3$, 6-$CF_3$ | $NO_2$ | H | $SO_2CH_3$ | $NO_2$ | |
| 534 | 3-$SO_2CH_3$, 6-$CF_3$ | $NO_2$ | H | $NO_2$ | $CF_3$ | |
| 535 | 3-$SO_2CH_3$, 6-$CF_3$ | $NO_2$ | H | $NO_2$ | Cl | |
| 536 | 3-$SO_2CH_3$, 6-$CF_3$ | CN | H | $NO_2$ | Cl | |
| 537 | 3-$SO_2CH_3$, 6-$NO_2$ | $NO_2$ | H | $CF_3$ | $NO_2$ | |
| 538 | 3-$SO_2CH_3$, 6-$NO_2$ | $NO_2$ | Cl | $CF_3$ | $NO_2$ | |
| 539 | 3-$SO_2CH_3$, 6-$NO_2$ | $NO_2$ | H | CN | $NO_2$ | |
| 540 | 3-$SO_2CH_3$, 6-$NO_2$ | $NO_2$ | Cl | Cl | $NO_2$ | |
| 541 | 3-$SO_2CH_3$, 6-$NO_2$ | $NO_2$ | H | $SO_2CH_3$ | $NO_2$ | |
| 542 | 3-$SO_2CH_3$, 6-$NO_2$ | $NO_2$ | H | $NO_2$ | $CF_3$ | |
| 543 | 3-$SO_2CH_3$, 6-$NO_2$ | $NO_2$ | H | $NO_2$ | Cl | |
| 544 | 3-$SO_2CH_3$, 6-$NO_2$ | CN | H | $NO_2$ | Cl | |
| 545 | 3-$SO_2CH_3$, 6-F | $NO_2$ | H | $CF_3$ | $NO_2$ | |
| 546 | 3-$SO_2CH_3$, 6-F | $NO_2$ | Cl | $CF_3$ | $NO_2$ | |
| 547 | 3-$SO_2CH_3$, 6-F | $NO_2$ | H | CN | $NO_2$ | |
| 548 | 3-$SO_2CH_3$, 6-F | $NO_2$ | Cl | Cl | $NO_2$ | |
| 549 | 3-$SO_2CH_3$, 6-F | $NO_2$ | H | $SO_2CH_3$ | $NO_2$ | |
| 550 | 3-$SO_2CH_3$, 6-F | $NO_2$ | H | $NO_2$ | $CF_3$ | |
| 551 | 3-$SO_2CH_3$, 6-F | $NO_2$ | H | $NO_2$ | Cl | |

Tabelle 2

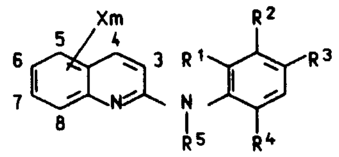

| Nr. | Xm | $R^1$ | $R^2$ | $R^3$ | $R^4$ | $R^5$ | Schmp. [°C] |
|---|---|---|---|---|---|---|---|
| 552 | 3-CN | $NO_2$ | H | $CF_3$ | $NO_2$ | $CH_3$ | |
| 553 | 3-CN | $NO_2$ | H | $CF_3$ | $NO_2$ | $COCH_3$ | |
| 554 | 3-CN | $NO_2$ | H | $CF_3$ | $NO_2$ | $SO_2C_6H_5$ | |
| 555 | 3-CN | $NO_2$ | Cl | $CF_3$ | $NO_2$ | $CH_3$ | 190-194 |
| 556 | 3-CN | $NO_2$ | Cl | $CF_3$ | $NO_2$ | $CH_2-CH=CH_2$ | |
| 557 | 3-CN | $NO_2$ | Cl | $CF_3$ | $NO_2$ | CHO | |
| 558 | 3-CN | $NO_2$ | Cl | $CF_3$ | $NO_2$ | $COCH_2Cl$ | |
| 559 | 3-CN | $NO_2$ | Cl | $CF_3$ | $NO_2$ | $COC_6H_5$ | |
| 560 | 3-CN | $NO_2$ | Cl | $CF_3$ | $NO_2$ | $SO_2C_6H_5$ | |
| 561 | 3-CN | $NO_2$ | H | $NO_2$ | $CF_3$ | $n-C_4H_9$ | |
| 562 | 3-CN | $NO_2$ | H | $NO_2$ | $CF_3$ | $CH_2-C\equiv CH$ | |
| 563 | 3-CN | $NO_2$ | H | $NO_2$ | $CF_3$ | $CH_2-C_6H_5$ | |
| 564 | 3-CN | $NO_2$ | H | $NO_2$ | $CF_3$ | $COCH_3$ | |
| 565 | 3-CN | $NO_2$ | H | $NO_2$ | $CF_3$ | $COCHCl_2$ | |
| 566 | 3-CN | $NO_2$ | H | $NO_2$ | $CF_3$ | $SO_2C_6H_5$ | |
| 567 | 3-CN | $NO_2$ | H | CN | $NO_2$ | $SO_2C_2H_5$ | |
| 568 | 3-CN | CN | H | $NO_2$ | Cl | $SO_2C_6H_5$ | |
| 569 | 3-CN, 6-$NO_2$ | $NO_2$ | Cl | $CF_3$ | $NO_2$ | $CH_3$ | |
| 570 | 3-CN, 6-$NO_2$ | $NO_2$ | Cl | $CF_3$ | $NO_2$ | $COCH_3$ | |
| 571 | 3-CN, 6-$NO_2$ | $NO_2$ | Cl | $CF_3$ | $NO_2$ | $SO_2C_6H_5$ | |
| 572 | 3-CN, 6-$NO_2$ | $NO_2$ | H | $NO_2$ | $CF_3$ | $SO_2C_6H_5$ | |
| 573 | 3-CN, 6-$NO_2$ | $NO_2$ | H | $NO_2$ | $CF_3$ | $CH_3$ | |
| 574 | 3-CN, 4-$CH_3$ | $NO_2$ | Cl | $CF_3$ | $NO_2$ | $SO_2C_6H_5$ | |
| 575 | 3-CN, 6-Cl | $NO_2$ | Cl | $CF_3$ | $NO_2$ | $SO_2C_6H_5$ | |
| 576 | 3-CN, 6-Cl | $NO_2$ | H | $NO_2$ | $CF_3$ | $SO_2C_6H_5$ | |
| 577 | 3-$CF_3$ | $NO_2$ | Cl | $CF_3$ | $NO_2$ | $SO_2C_6H_5$ | |
| 578 | 3-$CF_3$ | $NO_2$ | H | $NO_2$ | $CF_3$ | $SO_2C_6H_5$ | |
| 579 | 3-$CF_3$ | $NO_2$ | H | $NO_2$ | $CF_3$ | $CH_3$ | |
| 580 | 3-$CF_3$, 6-Cl | $NO_2$ | Cl | $CF_3$ | $NO_2$ | $CH_3$ | |
| 581 | 3-$CF_3$, 6-Cl | $NO_2$ | Cl | $CF_3$ | $NO_2$ | $COC_6H_5$ | |
| 582 | 3-$CF_3$, 6-Cl | $NO^2$ | Cl | $CF_3$ | $NO_2$ | $SO_2C_6H_5$ | |
| 583 | 3-$CF_3$, 6-Cl | $NO_2$ | H | $NO_2$ | $CF_3$ | $CH_2-C=CH$ | |
| 584 | 3-$CF_3$, 6-Cl | $NO_2$ | H | $NO_2$ | $CF_3$ | $COCCl_3$ | |
| 585 | 3-$CF_3$, 6-Cl | $NO_2$ | H | $NO_2$ | $CF_3$ | $SO_2C_6H_5$ | |
| 586 | 3-Cl, 6-$CF_3$ | $NO_2$ | Cl | $CF_3$ | $NO_2$ | $CH_2-CH=CH_2$ | |

Tabelle 2 - Forts.

| Nr. | Xm | $R^1$ | $R^2$ | $R^3$ | $R^4$ | $R^5$ | Schmp. [°C] |
|---|---|---|---|---|---|---|---|
| 587 | 3-Cl, 6-$CF_3$ | $NO_2$ | Cl | $CF_3$ | $NO_2$ | $COCHCl_2$ | |
| 588 | 3-Cl, 6-$CF_3$ | $NO_2$ | Cl | $CF_3$ | $NO_2$ | $SO_2C_6H_5$ | |
| 589 | 3-Cl, 6-$CF_3$ | $NO_2$ | H | $NO_2$ | $CF_3$ | $CH_3$ | |
| 590 | 3-Cl, 6-$CF_3$ | $NO_2$ | H | $NO_2$ | $CF_3$ | $COC_6H_5$ | |
| 591 | 3-Cl, 6-$CF_3$ | $NO_2$ | H | $NO_2$ | $CF_3$ | $SO_2C_6H_5$ | |
| 592 | 3-Cl, 4-$CH_3$, 6-F | $NO_2$ | Cl | $CF_3$ | $NO_2$ | $SO_2C_6H_5$ | |
| 593 | 3-Cl, 4-$CH_3$, 6-F | $NO_2$ | H | $NO_2$ | $CF_3$ | $SO_2C_6H_5$ | |
| 594 | 3-Cl, 4-$CH_3$, 6-F | CN | H | $NO_2$ | Cl | $SO_2$-$C_6H_5$ | |
| 595 | 3-Cl, 4-$CH_3$, 6-F | $NO_2$ | H | $NO_2$ | $CF_3$ | $CH_2$-C≡CH | |
| 596 | 3-Cl, 4-$CH_3$, 6-F | $NO_2$ | Cl | $CF_3$ | $NO_2$ | $CH_2$-CH=$CH_2$ | |
| 597 | 3-$CHF_2$ | $NO_2$ | Cl | $CF_3$ | $NO_2$ | $SO_2C_6H_5$ | |
| 598 | 3-$CHF_2$ | $NO_2$ | H | $NO_2$ | $CF_3$ | $SO_2C_6H_5$ | |
| 599 | 3-$CHF_2$ | $NO_2$ | H | $NO_2$ | Cl | $SO_2C_6H_5$ | |
| 600 | 3-$CHF_2$, 6-$CH_3$ | $NO_2$ | Cl | $CF_3$ | $NO_2$ | $SO_2C_6H_5$ | |
| 601 | 3-$CHF_2$, 6-$CH_3$ | $NO_2$ | H | $NO_2$ | $CF_3$ | $SO_2C_6H_5$ | |
| 602 | 3-Cl | $NO_2$ | Cl | $CF_3$ | $NO_2$ | $CH_3$ | |
| 603 | 3-Cl | $NO_2$ | Cl | $CF_3$ | $NO_2$ | $SO_2C_6H_5$ | |
| 604 | 3-Cl | $NO_2$ | H | $NO_2$ | $CF_3$ | $SO_2C_6H_5$ | |
| 605 | 3-Cl, 4-$CH_3$ | $NO_2$ | Cl | $CF_3$ | $NO_2$ | $SO_2C_6H_5$ | |
| 606 | 3-Cl, 4-$CH_3$ | $NO_2$ | H | $NO_2$ | $CF_3$ | $SO_2C_6H_5$ | |
| 607 | 3-Cl, 6-$CF_3$ | $NO_2$ | Cl | $CF_3$ | $NO_2$ | $SO_2C_6H_5$ | |
| 608 | 3-Cl, 6-$CF_3$ | $NO_2$ | H | $NO_2$ | $CF_3$ | $SO_2C_6H_5$ | |
| 609 | 3,6-$Cl_2$ | $NO_2$ | Cl | $CF_3$ | $NO_2$ | $SO_2C_6H_5$ | |
| 610 | 3,6-$Cl_2$ | $NO_2$ | H | $NO_2$ | $CF_3$ | $SO_2C_6H_5$ | |
| 611 | 3-Cl, 4-$CH_3$, 7-F | $NO_2$ | Cl | $CF_3$ | $NO_2$ | $SO_2C_6H_5$ | |
| 612 | 3-Cl, 4-$CH_3$, 7-F | $NO_2$ | H | $NO_2$ | $CF_3$ | $SO_2C_6H_5$ | |
| 613 | 3-$NO_2$, 6-$CF_3$ | $NO_2$ | Cl | $CF_3$ | $NO_2$ | $SO_2C_6H_5$ | |
| 614 | 3-$NO_2$, 6-$CF_3$ | $NO_2$ | H | $NO_2$ | $CF_3$ | $SO_2C_6H_5$ | |
| 615 | 3-$NO_2$, 6-Cl | $NO_2$ | Cl | $CF_3$ | $NO_2$ | $SO_2C_6H_5$ | |
| 616 | 3-$NO_2$, 6-Cl | $NO_2$ | H | $NO_2$ | $CF_3$ | $SO_2C_6H_5$ | |
| 617 | 3-$SO_2CH_3$ | $NO_2$ | Cl | $CF_3$ | $NO_2$ | $COCH_3$ | |
| 618 | 3-$SO_2CH_3$ | $NO_2$ | Cl | $CF_3$ | $NO_2$ | $SO_2C_6H_5$ | |
| 619 | 3-$SO_2CH_3$ | $NO_2$ | H | $NO_2$ | $CF_3$ | $SO_2C_6H_5$ | |
| 620 | 3-$SO_2CH_3$, 6-Cl | $NO_2$ | Cl | $CF_3$ | $NO_2$ | $SO_2C_6H_5$ | |
| 621 | 3-$SO_2CH_3$, 6-Cl | $NO_2$ | H | $NO_2$ | $CF_3$ | $SO_2C_6H_5$ | |
| 622 | 3-$SO_2CH_3$, 6-$NO_2$ | $NO_2$ | Cl | $CF_3$ | $NO_2$ | $SO_2C_6H_5$ | |
| 623 | 3-$SO_2CH_3$, 6-$NO_2$ | $NO_2$ | H | $NO_2$ | $CF_3$ | $SO_2C_6H_5$ | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Phycomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Phytophthora infestans an Tomaten und Kartoffeln,
Phytophthora parasitica an Erdbeeren,
Pseudoperonospora cubensis an Gurken,
Pseudoperonospora humuli an Hopfen,
Peronospora destructor an Zwiebeln,
Peronospora tabacina an Tabak,
Plasmopara viticola an Reben,
Plasmopara halstedii an Sonnenblumen,
Sclerospora macrospora an Mais,
Bremia lactucae an Salat,
Mucor mucedo an Früchten,
Rhizopus nigricans an Rüben sowie
Erysiphe graminis an Getreide,
Uncinula necator an Reben,
Sphaerotheca fuliginea an Kürbisgewächsen,
Puccinia-Arten an Getreide,
Rhizoctonia-Arten an Baumwolle,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis sowie
Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die neuen Verbindungen können auch im Materialschutz eingesetzt werden z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 26 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 39 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 26 werden in einer Mischung gelöst, die aus 40 Gew.-Teilen

Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 39 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 26 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 39 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 26 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 39 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 26 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde der bekannte Wirkstoff 2-(2,4-Dinitro-6-trifluormethylanilino)-5-trifluormethylpyridin (A) verwendet.

Anwendungsbeispiel 1

Wirksamkeit gegen Plasmopara viticola

Blätter von Topfreben der Sorte "Müller-Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24 °C und anschließend für 5 Tage in einem Gewächshaus mit Temperaturen zwischen 20 und 30 °C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis des Versuches zeigt, daß beispielsweise bei der Anwendung als 0,05 %ige (Gew.%) Spritzbrühe die Verbindungen 26 und 39 eine bessere fungizide Wirkung (97 %) haben als der bekannte Wirkstoff A (70 %).

Anwendungsbeispiel 2

Wirksamkeit gegen Septoria nodorum

Blätter von in Töpfen gewachsenen Weizenkeimlingen der Sorte "Jubilar" wurden mit wäßriger Spritzbrühe, die 80 % Wirkstoff und 20 % Emulgiermittel in der Trockensubstanz enthielt, bis zur

Tropfnässe besprüht. Am folgenden Tag wurden die Pflanzen mit einer wäßrigen Sporensuspension von Septoria nodorum infiziert und für 7 Tage bei 17 bis 19 °C und 95 % relativer Luftfeuchtigkeit weiter kultiviert. Das Ausmaß des Pilzbefalles wurde danach visuell ermittelt.

Das Ergebnis des Versuches zeigt, daß beispielsweise bei der Anwendung als 0,05 %ige Spritzbrühe die Verbindungen 26 und 39 eine bessere fungizide Wirkung (90 %) haben als der bekannte Wirkstoff A (50 %).

**Patentansprüche**

**1.** 2-Anilino-chinoline der Formel I,

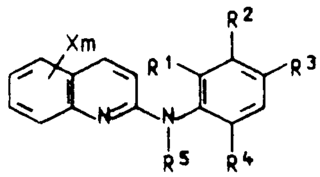

in welcher
X für Wasserstoff, $NO_2$, CN, Hal, $SO_2$-$C_1$-$C_4$-Alkyl, $C_1$-$C_4$-Alkyl, $C_1$-$C_4$-Haloalkyl steht,
m eine ganze Zahl von 1 - 6 ist, wobei die einzelnen Reste gleich oder verschieden sind, wenn m größer als 1 ist,
$R^1$, $R^3$, $R^4$ unabhängig voneinander Wasserstoff, $NO_2$, Hal, CN, $C_1$-$C_6$-Alkyl, $SO_2$-$C_1$-$C_4$-Alkyl, $C_1$-$C_6$-Haloalkyl, $SO_2NR^6R^7$ ist,
$R^2$ Wasserstoff, Hal, $C_1$-$C_4$-Haloalkyl ist,
$R^5$ Wasserstoff, $C_1$-$C_4$-Alkyl, CHO, $COR^8$, $SO_2R^8$, $C_3$-$C_6$-Alkenyl, $C_3$-$C_6$-Alkinyl, Benzyl ist,
$R^6$, $R^7$ unabhängig voneinander Wasserstoff, $C_1$- $C_4$-Alkyl ist,
$R^8$ $C_1$-$C_4$-Alkyl, $C_1$-$C_4$-Haloalkyl, Benzyl, Phenyl, Tolyl, Halogenphenyl, Nitrophenyl, Naphthyl ist,
und Salze der 2-Anilino-chinoline der Formel I, in der $R^5$ Wasserstoffbedeutet, mit pflanzenverträglichen Kationen, außer den Verbindungen, in denen $X_m$ H, $R^5$ H oder $CH_3$, $R^1$, $R^2$, $R^3$, $R^4$ H bedeuten, und außer der Verbindung, in der $X_m$ 4-$CH_3$, $R^5$, $R^1$, $R^2$, $R^3$, $R^4$ H bedeuten.

**2.** 2-(2,4-Dinitro-6-trifluormethylanilino)-3-cyanochinolin.

**3.** 2-(2,6-Dinitro-3-chlor-4-trifluormethylanilino)-3-cyanochinolin.

**4.** Fungizides Mittel, enthaltend eine fungizid wirksame Menge eines 2-Anilino-chinolins der Formel I

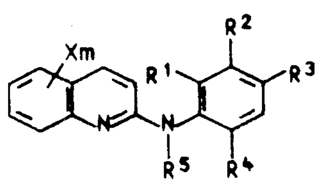

in welcher
X für Wasserstoff, $NO_2$, CN, Hal, $SO_2$-$C_1$-$C_4$-Alkyl, $C_1$-$C_4$-Alkyl, $C_1$-$C_4$-Haloalkyl steht,
m eine ganze Zahl von 1 - 6 ist, wobei die einzelnen Reste gleich oder verschieden sind, wenn m größer als 1 ist,
$R^1$, $R^3$, $R^4$ unabhängig voneinander Wasserstoff, $NO_2$, Hal, CN, $C_1$-$C_6$-Alkyl, $SO_2$-$C_1$-$C_4$-Alkyl, $C_1$-$C_6$-Haloalkyl, $SO_2NR^6R^7$ ist,
$R^2$ Wasserstoff, Hal, $C_1$-$C_4$-Haloalkyl ist,
$R^5$ Wasserstoff, $C_1$-$C_4$-Alkyl, CHO, $COR^8$, $SO_2R^8$, $C_3$-$C_6$-Alkenyl, $C_3$-$C_6$-Alkinyl, Benzyl ist,
$R^6$, $R^7$ unabhängig voneinander Wasserstoff, $C_1$- $C_4$-Alkyl ist,
$R^8$ $C_1$-$C_4$-Alkyl, $C_1$-$C_4$-Haloalkyl, Benzyl, Phenyl, Tolyl, Halogenphenyl, Nitrophenyl, Naphthyl ist,
oder eines Salzes eines 2-Anilino-chinolins der Formel I, in der $R^5$ Wasserstoff bedeutet, mit einem pflanzenverträglichen Kation außer den Verbindungen in denen $X_m$ H, $R_5$ H oder $CH_3$, $R_1$, $R_2$, $R_3$, $R_4$ H

bedeuten, und außer der Verbindung, in der $X_m$ 4-$CH_3$, $R_5$, $R_1$, $R_2$, $R_3$, $R_4$ H bedeuten, und einen festen oder flüssigen Trägerstoff.

**5.** Verfahren zur Bekämpfung von Fungi, dadurch gekennzeichnet, daß man eine fungizid wirksame Menge eines 2-Anilino-chinolins der Formel I

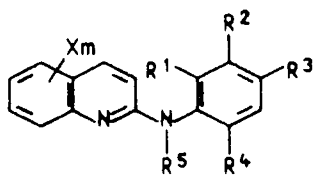

,

in welcher
X für Wasserstoff, $NO_2$, CN, Hal, $SO_2$-$C_1$-$C_4$-Alkyl, $C_1$-$C_4$-Alkyl, $C_1$-$C_4$-Haloalkyl steht,
m eine ganze Zahl von 1 - 6 ist, wobei die einzelnen Reste gleich oder verschieden sind, wenn m größer als 1 ist,
$R^1$, $R^3$, $R^4$ unabhängig voneinander Wasserstoff, $NO_2$, Hal, CN, $C_1$-$C_6$-Alkyl, $SO_2$-$C_1$-$C_4$-Alkyl, $C_1$-$C_6$-Haloalkyl, $SO_2NR^6R^7$ ist,
$R^2$ Wasserstoff, Hal, $C_1$-$C_4$-Haloalkyl ist,
$R^5$ Wasserstoff, $C_1$-$C_4$-Alkyl, CHO, $COR^8$, $SO_2R^8$, $C_3$-$C_6$-Alkenyl, $C_3$-$C_6$-Alkinyl, Benzyl ist,
$R^6$, $R^7$ unabhängig voneinander Wasserstoff, $C_1$- $C_4$-Alkyl ist,
$R^8$ $C_1$-$C_4$-Alkyl, $C_1$-$C_4$-Haloalkyl, Benzyl, Phenyl, Tolyl-Halogenphenyl, Nitrophenyl, Naphthyl ist,
oder eines Salzes eines 2-Anilino-chinolins der Formel I, in der $R^5$ Wasserstoff bedeutet, mit einem pflanzenverträglichen Kation auf Pilze oder durch Pilzbefall bedrohte Pflanzen, Saatgüter oder Materialien einwirken läßt.

**6.** 2-Anilino-chinolin gemäß Anspruch 1, dadurch gekennzeichnet, daß $X_m$ den Cyanrest in 3-Stellung, $R^1$ den Nitrorest, $R^2$ Wasserstoff oder Halogen und $R^4$ Nitro oder Trifluormethyl bedeuten.

**7.** 2-Anilino-chinolin gemäß Anspruch 1, dadurch gekennzeichnet, daß $X_m$ den Cyanrest in 3-Stellung, $R^1$ den Nitrorest, $R^2$ Wasserstoff oder Halogen und $R^3$ den Nitrorest bedeuten.

**8.** Verfahren zur Herstellung eines 2-Anilino-chinolins der Formel I

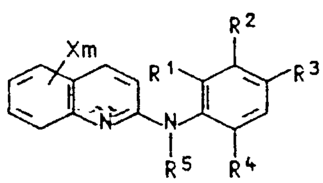

,

in welcher
X für Wasserstoff, $NO_2$, CN, Hal, $SO_2$-$C_1$-$C_4$-Alkyl, $C_1$-$C_4$-Alkyl, $C_1$-$C_4$-Haloalkyl steht,
m eine ganze Zahl von 1 - 6 ist, wobei die einzelnen Reste gleich oder verschieden sind, wenn m größer als 1 ist,
$R^1$, $R^3$, $R^4$ unabhängig voneinander Wasserstoff, $NO_2$, Hal, CN, $C_1$-$C_6$-Alkyl, $SO_2$-$C_1$-$C_4$-Alkyl, $C_1$-$C_6$-Haloalkyl, $SO_2NR^6R^7$ ist,
$R^2$ Wasserstoff, Hal, $C_1$-$C_4$-Haloalkyl ist,
$R^5$ Wasserstoff, $C_1$-$C_4$-Alkyl, CHO, $COR^8$, $SO_2R^8$, $C_3$-$C_6$-Alkenyl, $C_3$-$C_6$-Alkinyl, Benzyl ist,
$R^6$, $R^7$ unabhängig voneinander Wasserstoff, $C_1$-$C_4$-Alkyl ist,
$R^8$ $C_1$-$C_4$-Alkyl, $C_1$-$C_4$-Haloalkyl, Benzyl, Phenyl, Tolyl, Halogenphenyl, Nitrophenyl, Naphthyl ist, außer den Verbindungen, in den $X_m$ H, $R^5$ H oder $CH_3$, $R^1$, $R^2$, $R^3$, $R^4$ H bedeuten, und außer der Verbindung, in der $X_m$ 4-$CH_3$, $R^5$, $R^1$, $R^2$, $R^3$, $R^4$ H bedeuten,

dadurch gekennzeichnet, daß man ein Chinolin der Formel II, in der X und m die oben angegebene

Bedeutung haben, mit Verbindungen der Formel III

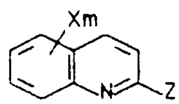

II

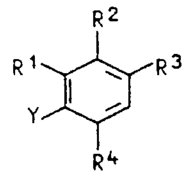

III

,

in der $R^1$, $R^2$, $R^3$, $R^4$ die oben angegebene Bedeutung haben, in Substanz oder in einem Lösungs- oder Verdünnungsmittel bei Temperaturen zwischen -20 und 200 °C umsetzt, wobei Y und Z für $NH_2$ oder Halogen stehen und Y und Z jeweils voneinander verschieden sind und gegebenenfalls ein Chinolin der Formel I, in der $R^5$ Wasserstoff ist und X, m, $R^1$, $R^2$, $R^3$, $R^4$, $R^6$, $R^7$ die oben angegebene Bedeutung haben, mit einem Alkylierungs-, Acylierungs- oder Sulfonylierungsmittel der Formel IV

$LR^5$ IV ,

in der
$R^5$ die oben angegebene Bedeutung außer Wasserstoff hat und L für eine nucleophil verdrängbare Abgangsgruppe steht, in Substanz oder in Gegenwart eines Lösungs- oder Verdünnungsmittels bei Temperaturen zwischen -20 °C und 150 °C umsetzt.

## Claims

**1.** A 2-anilinoquinoline of the formula I

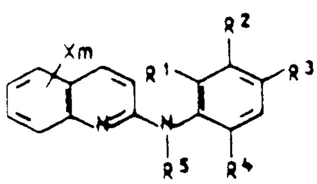

where
X is hydrogen, $NO_2$, CN, Hal, $SO_2$-$C_1$-$C_4$-alkyl, $C_1$-$C_4$-alkyl, $C_1$-$C_4$-haloalkyl,
m is an integer of from 1 to 6, the individual radicals being identical or different when m is greater than 1,
$R^1$, $R^3$ and $R^4$ independently of one another are each hydrogen, $NO_2$, Hal, CN, $C_1$-$C_6$-alkyl, $SO_2$-$C_1$-$C_4$-alkyl, $C_1$-$C_6$-haloalkyl or $SO_2NR^6R^7$,
$R^2$ is hydrogen, Hal or $C_1$-$C_4$-haloalkyl,
$R^5$ is hydrogen, $C_1$-$C_4$-alkyl, CHO, $COR^8$, $SO_2R^8$, $C_3$-$C_6$-alkenyl, $C_3$-$C_6$-alkynyl or benzyl,
$R^6$ and $R^7$ independently of one another are each hydrogen or $C_1$-$C_4$-alkyl and
$R^8$ is $C_1$-$C_4$-alkyl, $C_1$-$C_4$-haloalkyl, benzyl, phenyl, tolyl, halophenyl, nitrophenyl or naphthyl,
and salts of the 2-anilinoquinoline of the formula I where $R^5$ is hydrogen with plant-tolerated cations, except for the compounds in which $X_m$ is H, $R^5$ is H or $CH_3$ and $R^1$, $R^2$, $R^3$ and $R^4$ are each H and except for the compound in which $X_m$ is 4-$CH_3$ and $R^5$, $R^1$, $R^2$, $R^3$ and $R^4$ are each H.

**2.** 2-(2,4-dinitro-6-trifluoromethylanilino)-3-cyanoquinoline.

**3.** 2-(2,6-dinitro-3-chloro-4-trifluoromethylanilino)-3-cyanoquinoline.

**4.** A fungicide containing a fungicidal amount of a 2-anilinoquinoline of the formula I

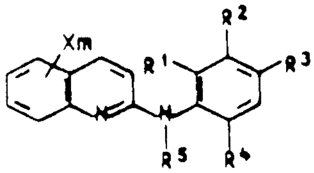

where
X is hydrogen, $NO_2$, CN, Hal, $SO_2$-$C_1$-$C_4$-alkyl, $C_1$-$C_4$-alkyl, $C_1$-$C_4$-haloalkyl,
m is an integer of from 1 to 6, the individual radicals being identical or different when m is greater than 1,
$R^1$, $R^3$ and $R^4$ independently of one another are each hydrogen, $NO_2$, Hal, CN, $C_1$-$C_6$-alkyl, $SO_2$-$C_1$-$C_4$-alkyl, $C_1$-$C_6$-haloalkyl or $SO_2NR^6R^7$,
$R^2$ is hydrogen, Hal or $C_1$-$C_4$-haloalkyl,
$R^5$ is hydrogen, $C_1$-$C_4$-alkyl, CHO, $COR^8$, $SO_2R^8$, $C_3$-$C_6$-alkenyl, $C_3$-$C_6$-alkynyl or benzyl,
$R^6$ and $R^7$ independently of one another are each hydrogen or $C_1$-$C_4$-alkyl and
$R^8$ is $C_1$-$C_4$-alkyl, $C_1$-$C_4$-haloalkyl, benzyl, phenyl, tolyl, halophenyl, nitrophenyl or naphthyl,
or a salt of a 2-anilinoquinoline of the formula I where $R^5$ is hydrogen with a plant-tolerated cation, except for the compounds in which $X_m$ is H, $R^5$ is H or $CH_3$ and $R^1$, $R^2$, $R^3$ and $R^4$ are each H and except for the compound in which $X_m$ is 4-$CH_3$ and $R^5$, $R^1$, $R^2$, $R^3$ and $R^4$ are each H, and a solid or liquid carrier.

**5.** A method for controlling fungi, wherein a fungicidal amount of a 2-anilinoquinoline of the formula I

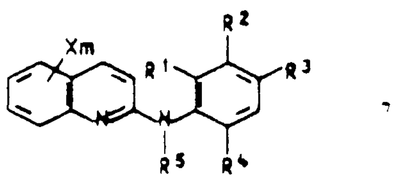

where
X is hydrogen, $NO_2$, CN, Hal, $SO_2$-$C_1$-$C_4$-alkyl, $C_1$-$C_4$-alkyl, $C_1$-$C_4$-haloalkyl,
m is an integer of from 1 to 6, the individual radicals being identical or different when m is greater than 1,
$R^1$, $R^3$ and $R^4$ independently of one another are each hydrogen, $NO_2$, Hal, CN, $C_1$-$C_6$-alkyl, $SO_2$-$C_1$-$C_4$-alkyl, $C_1$-$C_6$-haloalkyl or $SO_2NR^6R^7$,
$R^2$ is hydrogen, Hal or $C_1$-$C_4$-haloalkyl,
$R^5$ is hydrogen, $C_1$-$C_4$-alkyl, CHO, $COR^8$, $SO_2R^8$, $C_3$-$C_6$-alkenyl, $C_3$-$C_6$-alkynyl or benzyl,
$R^6$ and $R^7$ independently of one another are each hydrogen or $C_1$-$C_4$-alkyl and
$R^8$ is $C_1$-$C_4$-alkyl, $C_1$-$C_4$-haloalkyl, benzyl, phenyl, tolyl, halophenyl, nitrophenyl or naphthyl,
or a salt of a 2-anilinoquinoline of the formula I where $R^5$ is hydrogen with a plant-tolerated cation, is allowed to act on fungi or on plants, seeds or materials threatened by fungal attack.

**6.** The 2-anilinoquinoline as claimed in claim 1, wherein $X_m$ is cyano in the 3-position, $R^1$ is nitro, $R^2$ is hydrogen or halogen and $R^4$ is nitro or trifluoromethyl.

**7.** The 2-anilinoquinoline as claimed in claim 1, wherein $X_m$ is cyano in the 3-position, $R^1$ and $R^3$ are each nitro and $R^2$ is hydrogen or halogen.

**8.** A process for the preparation of a 2-anilinoquinoline of the formula I

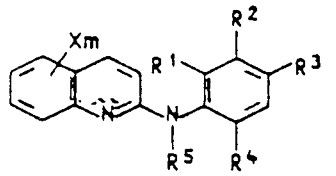

,

where
X is hydrogen, $NO_2$, CN, Hal, $SO_2$-$C_1$-$C_4$-alkyl, $C_1$-$C_4$-alkyl, $C_1$-$C_4$-haloalkyl,
m is an integer of from 1 to 6, the individual radicals being identical or different when m is greater than 1,
$R^1$, $R^3$ and $R^4$ independently of one another are each hydrogen, $NO_2$, Hal, CN, $C_1$-$C_6$-alkyl, $SO_2$-$C_1$-$C_4$-alkyl, $C_1$-$C_6$-haloalkyl or $SO_2NR^6R^7$,
$R^2$ is hydrogen, Hal or $C_1$-$C_4$-haloalkyl,
$R^5$ is hydrogen, $C_1$-$C_4$-alkyl, CHO, $COR^8$, $SO_2R^8$, $C_3$-$C_6$-alkenyl, $C_3$-$C_6$-alkynyl or benzyl,
$R^6$ and $R^7$ independently of one another are each hydrogen or $C_1$-$C_4$-alkyl and
$R^8$ is $C_1$-$C_4$-alkyl, $C_1$-$C_4$-haloalkyl, benzyl, phenyl, tolyl, halophenyl, nitrophenyl or naphthyl,
except for the compounds in which $X_m$ is H, $R^5$ is H or $CH_3$ and $R^1$, $R^2$, $R^3$ and $R^4$ are each H and except for the compound in which $X_m$ is 4-$CH_3$ and $R^5$, $R^1$, $R^2$, $R^3$ and $R^4$ are H,
wherein a quinoline of the formula II where X and m have the abovementioned meanings is reacted with a compound of the formula III

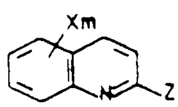

II

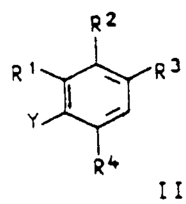

,

III

where $R^1$, $R^2$, $R^3$ and $R^4$ have the abovementioned meanings, in the presence or absence of a solvent or diluent at from -20 to 200 °C, Y and Z being $NH_2$ or halogen and Y and z being different from one another, and, if required, a quinoline of the formula I, where $R^5$ is hydrogen and X, m, $R^1$, $R^2$, $R^3$, $R^4$, $R^6$ and $R^7$ have the abovementioned meanings, is reacted with an alkylating, acylating or sulfonylating agent of the formula IV,

$LR^5$ IV,

where
$R^5$ has the abovementioned meanings except for hydrogen and L is a nucleophilically displaceable leading group, in the presence or absence of a solvent or diluent at from -20 to 150 °C.

## Revendications

**1.** 2-Anilino-quinoléines de formule I

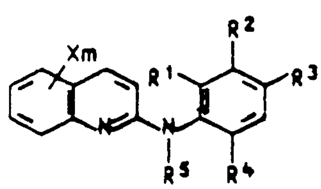

dans laquelle

X est mis pour hydrogène, $NO_2$, CN, hal, $SO_2$-alkyle en C 1-C 4, alkyle en C 1-C 4, haloalkyle en C 1-C 4,

m représente un nombre entier de 1 à 6, les différents restes étant identiques ou différents lorsque m est supérieur à 1,

$R^1$, $R^3$, $R^4$, indépendamment les uns des autres, représentent hydrogène, $NO_2$, hal, CN, alkyle en C 1-C 6, $SO_2$-alkyle en C 1-C 4, haloalkyle en C 1-C 6,

$SO_2NR^6R^7$,

$R^2$ représente hydrogène, hal, haloalkyle en C 1-C 4,

$R^5$ représente hydrogène, alkyle en C 1-C 4, CHO, $COR^8$, $SO_2R^8$, alcényle en C 3-C 6, alcynyle en C 3-C 6, benzyle,

$R^6$, $R^7$, indépendamment l'un de l'autre, représentent hydrogène, alkyle en C 1-C 4,

$R^8$ étant alkyle en C 1-C 4, haloalkyle en C 1-C 4, benzyle, phényle, tolyle, halogénophényle, nitrophényle, naphtyle,

et sels des 2-anilino-quinoléines de formule I, dans laquelle

$R^5$ représente hydrogène, avec des cations compatibles avec les plantes, excepté les composés dans lesquels $X_m$ représente H, $R^5$H ou $CH_3$, $R^1$, $R^2$, $R^3$, $R^4$ signifiant H, et excepté le composé dans lequel $X_m$ représente 4-$CH_3$, $R^5$, $R^1$, $R^2$, $R^3$ et $R^4$ étant mis pour H.

**2.** 2-(2,4-Dinitro-6-trifluorométhylanilino)-3-cyano-quinoléine.

**3.** 2-(2,6-Dinitro-3-chloro-4-trifluorométhylanilino)-3-cyanoquinoléine.

**4.** Agent fongicide contenant une quantité efficace au point de vue fongicide d'une 2-anilino-quinoléine de formule I

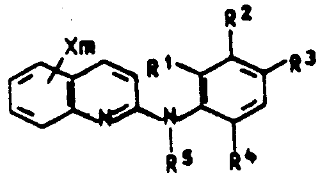

dans laquelle

X est mis pour hydrogène, $NO_2$, CN, hal, $SO_2$-alkyle en C 1-C 4, alkyle en C 1-C 4, haloalkyle en C 1-C 4,

m est un nombre entier de 1 à 6, les différents restes étant identiques ou différents lorsque m est supérieur à 1,

$R^1$, $R^3$, $R^4$, indépendamment les uns des autres, représentent hydrogène, $NO_2$, hal, CN, alkyle en C 1-C 6, $SO_2$-alkyle en C 1-C 4, haloalkyle en C 1-C 6,

$SO_2NR^6R^7$,

$R^2$ représente hydrogène, hal, haloalkyle en C 1-C 4,

$R^5$ représente hydrogène, alkyle en C 1-C 4, CHO, $COR^8$, $SO_2R^8$, alcényle en C 3-C 6, alcynyle en C 3-C 6, benzyle,

$R^6$, $R^7$, indépendamment l'un de l'autre, représentent hydrogène, alkyle en C 1-C 4,

$R^8$ étant alkyle en C 1-C 4, haloalkyle en C 1-C 4, benzyle, phényle, tolyle, halogénophényle, nitrophényle, naphtyle,

ou d'un sel d'une 2-anilino-quinoléine de formule I, dans laquelle $R^5$ représente hydrogène, avec un cation toléré par les plantes, excepté les composés dans lesquels $X_m$ représente H, $R^5$H ou $CH_3$, $R^1$, $R^2$, $R^3$, $R^4$ signifiant H, et excepté le composé dans lequel $X_m$ représente 4-$CH_3$, $R^5$, $R^1$, $R^2$, $R^3$ et $R^4$ étant mis pour H, et un véhicule solide ou liquide.

**5.** Procédé de lutte contre les champignons, caractérisé par le fait que l'on laisse agir sur les champignons ou les plantes, semences ou matériaux menacés par l'attaque par les champignons, une quantité efficace du point de vue fongicide d'une 2-anilino-quinoléine de formule I

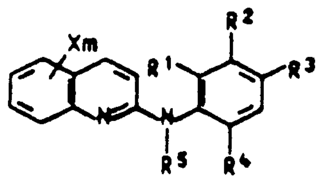

dans laquelle

X est mis pour hydrogène, $NO_2$, CN, hal, $SO_2$-alkyle en C 1-C 4, alkyle en C 1-C 4, haloalkyle en C 1-C 4,

m est un nombre entier allant de 1 à 6, les différents restes étant identiques ou différents losque m est supérieur à 1,

$R^1$, $R^3$, $R^4$, indépendamment les uns des autres, représentent hydrogène, $NO_2$, hal, CN, alkyle en C 1-C 6, $SO_2$-alkyle en C 1-C 4, haloalkyle en C 1-C 6,

$SO_2NR^6R^7$,

$R^2$ représente hydrogène, hal, haloalkyle en C 1-C 4,

$R^5$ représente hydrogène, alkyle en C 1-C 4, CHO, $COR^8$, $SO_2R^8$, alcényle en C 3-C 6, alcynyle en C 3-C 6, benzyle,

$R^6$, $R^7$, indépendamment l'un de l'autre, représentent hydrogène, alkyle en C 1-C 4,

$R^8$ étant alkyle en C 1-C 4, haloalkyle en C 1-C 4, benzyle, phényle, tolyle, halogénophényle, nitrophényle, naphtyle,

ou d'un sel d'une 2-anilino-quinoléine de formule I, dans laquelle $R^5$ représente hydrogène, avec un cation toléré par les plantes.

**6.** 2-Anilino-quinoléine selon la revendication 1, caractérisée par le fait que $X_m$ représente le reste cyano en position 3, $R^1$ le reste nitro, $R^2$ hydrogène ou halogène et $R^4$ nitro ou trifluorométhyle.

**7.** 2-Anilino-quinoléine selon la revendication 1, caractérisée par le fait que $X_m$ représente le reste cyano en position 3, $R^1$ le reste nitro, $R^2$ hydrogène ou halogène et $R^3$ le reste nitro.

**8.** Procédé de préparation d'une 2-anilino-quinoléine de formule I

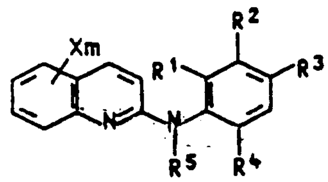

dans laquelle

X est mis pour hydrogène, $NO_2$, CN, hal, $SO_2$-alkyle en C 1-C 4, alkyle en C 1-C 4, haloalkyle en C 1-C 4,

m est un nombre entier allant de 1 à 6, les différents restes étant identiques ou différents lorsque m est supérieur à 1,

$R^1$, $R^3$, $R^4$, indépendamment les uns des autres, représentent hydrogène, $NO_2$, hal, CN, alkyle en C 1-C 6, $SO_2$-alkyle en C 1-C 4, haloalkyle en C 1-C 6,

$SO_2NR^6R^7$,

$R^2$ représente hydrogène, hal, haloalkyle en C 1-C 4,

$R^5$ représente hydrogène, alkyle en C 1-C 4, CHO, $COR^8$, $SO_2R^8$, alcényle en C 3-C 6, alcynyle en C 3-C 6, benzyle,

$R^6$, $R^7$, indépendamment l'un de l'autre, représentent hydrogène, alkyle en C 1-C 4,

$R^8$ étant alkyle en C 1-C 4, haloalkyle en C 1-C 4, benzyle, phényle, tolyle, halogénophényle, nitrophényle, naphtyle, excepté les composés dans lesquels $X_m$ représente H, $R^5$H ou $CH_3$, $R^1$, $R^2$, $R^3$, $R^4$ signifiant H, et excepté le composé dans lequel $X_m$ représente 4-$CH_3$, $R^5$, $R^1$, $R^2$, $R^3$ et $R^4$ étant mis pour H, caractérisé par le fait que l'on met à réagir, à des températures comprises entre -20 et 200 degrés C, une quinoléine de formule II, dans laquelle X et m ont les significations données plus haut, avec des composés de formule III

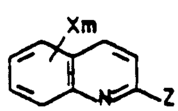

II

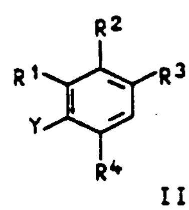

III

dans laquelle $R^1$, $R^2$, $R^3$, $R^4$ ont les significations données plus haut, comme tels ou dans un solvant ou diluant, Y et Z étant mis pour $NH_2$ ou halogène et étant dans chaque cas différents l'un de l'autre, et éventuellement on fait réagir, à des températures comprises entre -20 et 150 degrés C, une quinoléine de formule I, dans laquelle $R^5$ représente hydrogène et X, m, $R^1$, $R^2$, $R^3$, $R^4$, $R^6$, $R^7$ ont les significations données plus haut, avec un agent d'alkylation, d'acylation ou de sulfonylation, - comme tel ou en présence d'un solvant ou diluant - de formule IV

$LR^5$ IV

dans laquelle

$R^5$ a la signification donnée plus haut, excepté hydrogène, L représentant un groupe de départ déplaçable par un réactif nucléophile.